# EUROPEAN PATENT APPLICATION

(11) **EP 4 725 488 A1**
(43) Date of publication of application: **15.04.2026**
(21) Application number: 24819627.1
(22) Date of filing: 07.06.2024
(51) Int. Cl.: A61K 31/506, A61K 45/06, A61P 35/00, C07D 401/12

(54) **PHARMACEUTICAL COMPOSITION FOR PREVENTING OR TREATING BRAIN TUMOR**

(30) Priority: 09.06.2023 KR 20230073989; 05.06.2024 KR 20240074033
(71) Applicant: Astrion Inc., Seoul 02842 (KR)
(72) Inventor: JEONG, Geuk-Rae, Seoul 02842 (KR); PARK, Hee Jo, Seoul 02842 (KR)
(74) Representative: Nederlandsch Octrooibureau
(86) International application number: PCT/KR2024/007833
(87) International publication number: WO 2024/253476

(57) **Abstract**

The present invention relates to the use of a novel anoctamin 1 (ANO1) inhibitory compound for preventing and treating brain tumors, and is completed by confirming that the compound can inhibit brain tumors by inhibiting the expression or activity of ANO1, which is a calcium-dependent chloride channel. The growth, migration, and proliferation of brain tumor cells treated with the compound were significantly inhibited, and such inhibitory effects were shown to be superior to conventional ANO1 inhibitors. In particular, the compound of the present invention can simultaneously inhibit ANO1 and EGFR in brain tumor cells, thereby exhibiting a strong anticancer effect through dual inhibition of the two proteins, further enhancing the efficacy of an anticancer agent when used in combination with an EGFR targeted anticancer agent, and inhibiting cancer resistance. Therefore, the compound itself can be used as a dual target anticancer agent of ANO1 and EGFR, and can also be used as a combination agent of EGFR targeted therapy, and thus is expected to be variously utilized in the field of prevention and treatment of brain tumors.

## Description

### [Technical Field]

The present invention relates to a pharmaceutical composition for preventing, improving, or treating brain tumor, which comprises, as an active ingredient, a novel compound capable of simultaneously inhibiting ANO1 (Anoctamin 1) and EGFR (epidermal growth factor receptor).

This application claims priority to and the benefit of Korean Patent Application No. 10-2023-0073989, filed on June 09, 2023, and Korean Patent Application No. 10-2024-0074033, filed on June 05, 2024, the disclosure of which is incorporated herein by reference in its entirety.

### [Background Art]

Glioblastoma is a primary malignant brain tumor that occurs in adults. If left untreated after diagnosis, it is a highly malignant disease that causes death within 3 to 6 months, and it has an average survival period of only 12 to 14 months even when treated using all kinds of available treatment methods. In South Korea, approximately 12,000 people develop brain tumors every year. Among them, approximately 9,000 are diagnosed with benign tumors, such as meningiomas or pituitary adenomas, and approximately 2,000 are diagnosed with malignant tumors. Approximately 630 patients with malignant tumors are diagnosed with glioblastoma each year.

Because glioblastoma, which originates from glial cells in the brain tissue, grows invasively into the surrounding brain tissue, it has unclear tumor boundaries and extends farther than the actual visual boundaries seen with the naked eye during radiological examinations or surgeries. Glioblastoma is often located in areas of the brain where surgical removal is impossible due to functional reasons, and complete remission is not achieved with radiation therapy. Furthermore, glioblastoma is an incurable disease with a poor prognosis, making treatment very difficult due to the lack of effective anticancer agents, as the anticancer agents do not easily penetrate the brain due to the blood-brain barrier. Temozolomide, which is the current standard treatment for glioblastoma, is used, but long-term treatment may cause side effects and resistance, making sustained treatment difficult. Despite many modern medical advancements and new drug developments, no new treatment has yet been developed.

One of the oncogenes that frequently undergo genetic alterations in patients with glioblastoma is an epidermal growth factor receptor (EGFR). EGFR is physiologically important, and mutations in EGFR are observed in more than half of such patients. When EGFR binds to EGF or TNF-alpha, various signaling events occur within the cell. However, overexpression or hyperactive mutations of EGFR cause excessive cell proliferation, which ultimately leads to cancer. Among these EGFR mutations, EGFRvIII is a protein whose mutation is characterized by the deletion of amino acids 6 to 273, and is specifically expressed in cancer cells. While EGFRvIII was first discovered in glioblastoma, it has also been found in other tissue cancers.

It has been reported that volume control of cancer cells by chloride ion channel proteins such as Anoctamin 1 (ANO1) and the chloride channel (CLC) family is important in the migration and metastasis of glioblastoma cells. ANO1 proteins are involved in various physiological functions (pain control, blood pressure control, brain nerve development, water secretion control, and the like). In particular, their anticancer effects have been reported in various cancer cells (breast cancer, head and neck cancer, prostate cancer, pancreatic cancer, lung cancer, and the like). Inhibition of ANO1 proteins in breast cancer exerted anticancer effects through CaMK2 signaling. Also, it has been reported that although the level of ANO1 expression in the normal brain is significantly low, the ANO1 proteins are overexpressed in glioblastoma, and inhibition of ANO1 activity inhibits cell migration and metastasis in glioblastoma, thereby suppressing cancer growth in animal models. Furthermore, it has been reported that inhibition of ANO1 in glioblastoma stem cells induces the epidermal growth factor receptor variant 3 (EGFRvIII) protein degradation. These studies suggest that inhibition of ANO1 proteins could be a therapeutic strategy for various EGFR mutation-associated cancers, including glioblastoma.

Thus, both ANO1 and EGFR have potential as anticancer therapeutic targets. However, no therapeutic drugs capable of exhibiting superior anticancer effects through dual inhibition of ANO1 and EGFR have yet been found.

### [Disclosure]

### [Technical Problem]

The present invention relates to the use of novel Anoctamin 1 (ANO1) inhibitory compounds for the prevention and treatment of brain tumor, and was completed by confirming that the compounds can simultaneously inhibit ANO1, a calcium-dependent chloride channel, and EGFR, a tyrosine kinase, thereby exhibiting excellent anticancer effects.

Therefore, an object of the present invention is to provide one or more compounds selected from the group consisting of the compounds represented by Chemical Formulae 1 to 6, or pharmaceutically acceptable salts thereof.

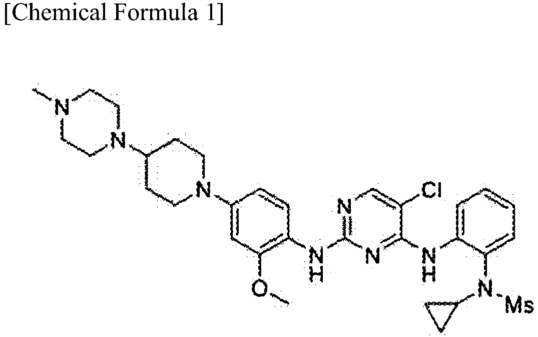

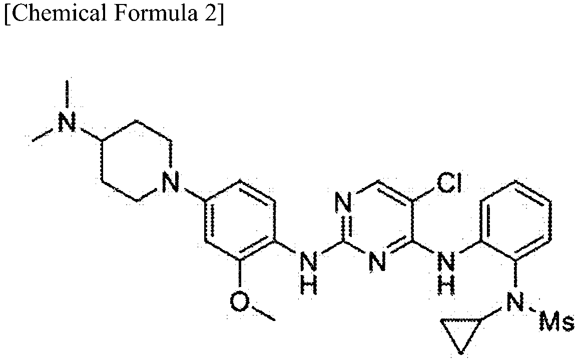

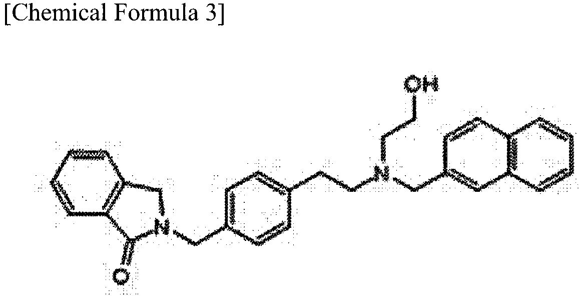

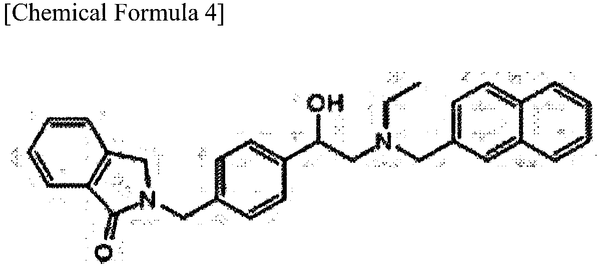

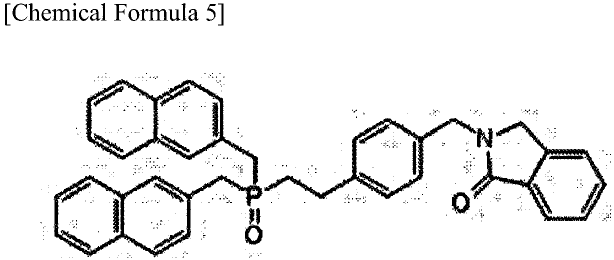

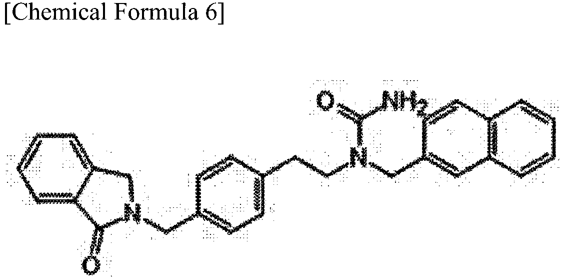

Another object of the present invention is to provide a method for preparing the compounds represented by Chemical Formulae 1 to 6.

Still another object of the present invention is to provide a pharmaceutical composition for the prevention or treatment of brain tumors, comprising as an active ingredient at least one compound selected from the group consisting of the compounds represented by Chemical Formulae 1 to 6 or pharmaceutically acceptable salts thereof.

Still another object of the present invention is to provide a pharmaceutical composition for the prevention or treatment of brain tumors, comprising as active ingredients (i) at least one compound selected from the group consisting of the compounds represented by Chemical Formulae 1 to 6 or pharmaceutically acceptable salts thereof; and (ii) a targeted anticancer agent against EGFR.

Yet another object of the present invention is to provide a pharmaceutical composition for enhancing the anticancer effect of a brain tumor anticancer agent, comprising as an active ingredient at least one compound selected from the group consisting of the compounds represented by Chemical Formulae 1 to 6 or pharmaceutically acceptable salts thereof.

However, technical problems to be solved in the present invention are not limited to the above-described problems, and other problems which are not described herein will be fully understood by those of ordinary skill in the art from the following descriptions.

### [Technical Solution]

In order to achieve the above object, the present invention provides one or more compounds selected from the group consisting of the compounds represented by Chemical Formulae 1 to 6, or pharmaceutically acceptable salts thereof:

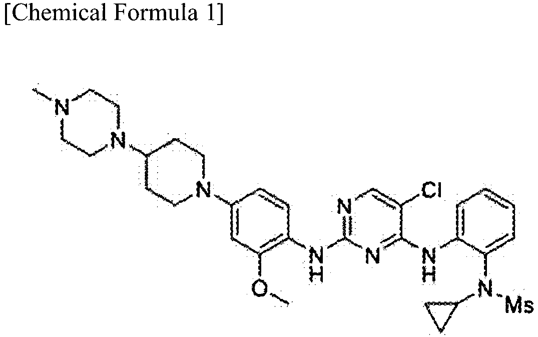

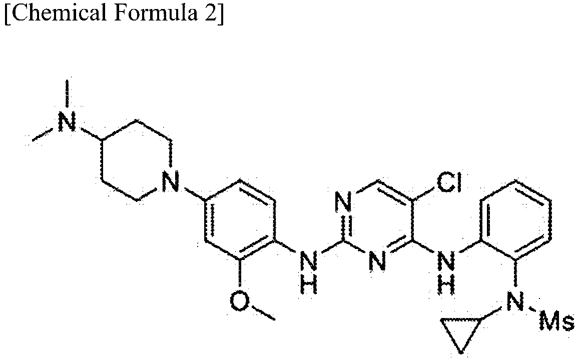

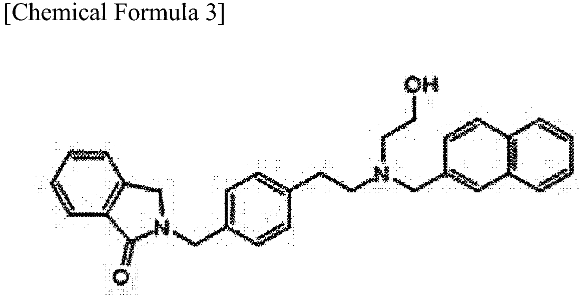

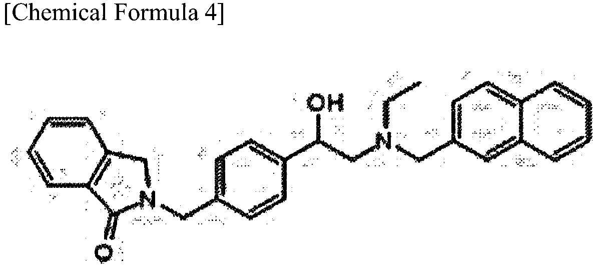

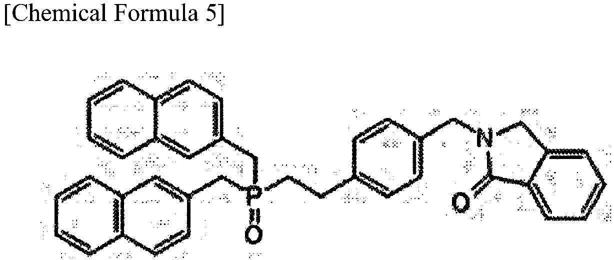

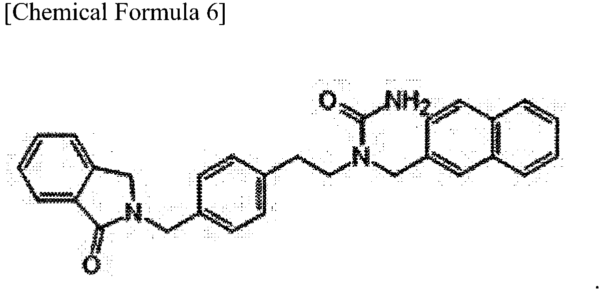

In addition, the present invention provides a method for preparing the compounds represented by Chemical Formulae 1 to 6, comprising:
(S1A) preparing a compound represented by the following Chemical Formula 8 by allowing a compound represented by the following Chemical Formula 7 to react with methanesulfonyl chloride (MsCl) and cyclopropyl amine in the presence of a base; or
(S1B) allowing a compound represented by the following Chemical Formula 9 to react with a compound represented by the following Chemical Formula 10 or 11 in the presence of a base.

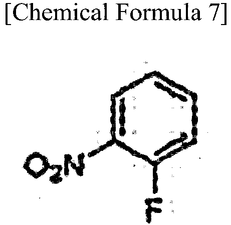

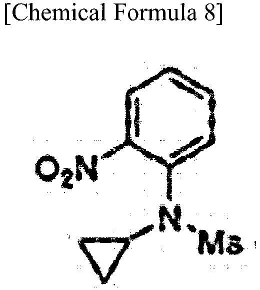

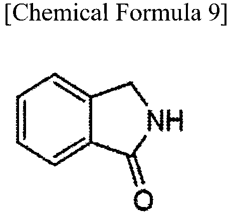

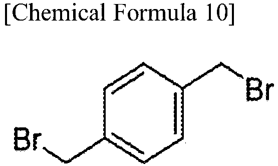

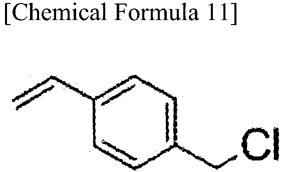

In one embodiment of the present invention, the step (S1A) may further comprise the following steps, but is not limited thereto:
(S1A-1) reducing the compound (the nitro group) represented by Chemical Formula 8 with zinc in the presence of ammonium chloride to prepare a compound represented by Chemical Formula 12;
(S1A-2) allowing the compound represented by Chemical Formula 12 to react with 2,4,5-trichloropyrimidine in the presence of a base to prepare a compound represented by Chemical Formula 13; and
(S1A-3) allowing the compound represented by Chemical Formula 13 to react with a compound represented by Chemical Formula 14 or Chemical Formula 15 in the presence of an acid to prepare a compound represented by Chemical Formula 1 or Chemical Formula 2.

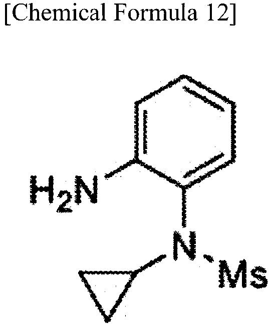

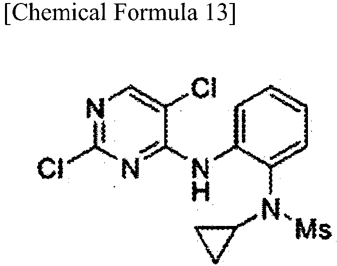

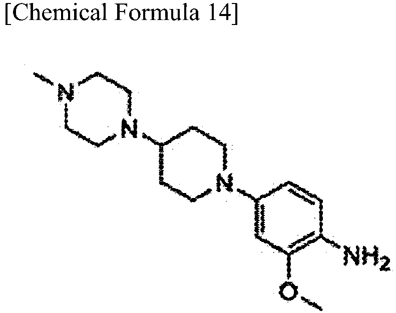

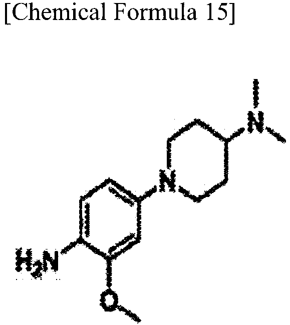

In another embodiment of the present invention, a compound represented by the following Chemical Formula 16 is prepared when the compound represented by Chemical Formula 9 is allowed to react with the compound represented by Chemical Formula 10 in the presence of a base in the step (S1B), but is not limited thereto.

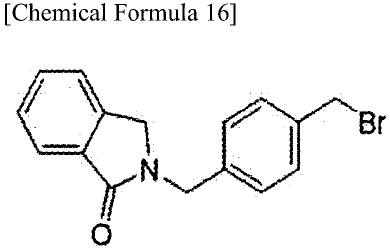

In yet another embodiment of the present invention, the step (S1B) may further comprise the following steps, but is not limited thereto:
(S1B-1) allowing the compound represented by Chemical Formula 16 to react with trimethylsilyl cyanide in the presence of a base to prepare a compound represented by the following Chemical Formula 17;
(S1B-2) hydrogenating the compound represented by Chemical Formula 17 in the presence of a metal catalyst to prepare a compound represented by the following Chemical Formula 18;
(S1B-3) allowing the compound represented by Chemical Formula 18 to react with a compound represented by the following Chemical Formula 19 in the presence of a reducing agent to prepare a compound represented by the following Chemical Formula 20; and
(S1B-4) allowing the compound represented by Chemical Formula 20 to react with NCO- to prepare the compound represented by Chemical Formula 6; or allowing the compound represented by Chemical Formula 20 to react with a compound represented by the following Chemical Formula 21 in the presence of a base to prepare a compound represented by the following Chemical Formula 22.

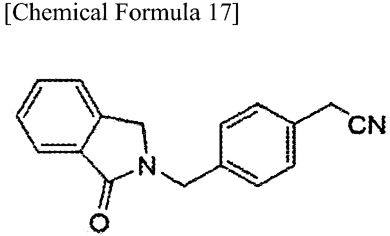

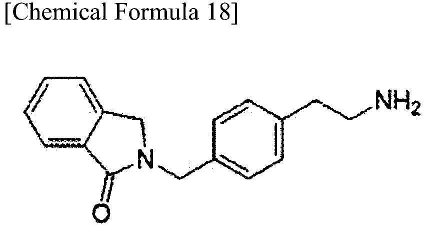

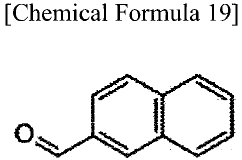

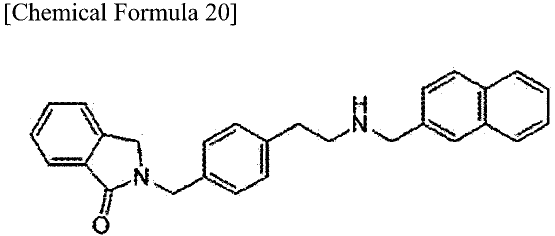

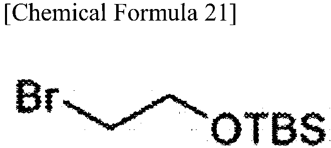

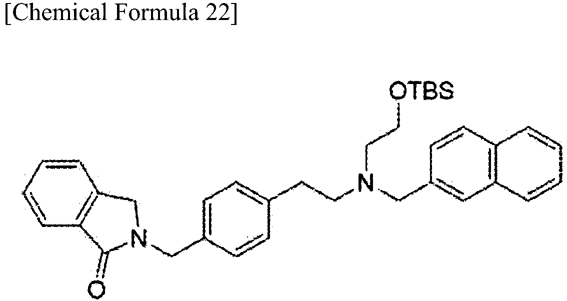

In yet another embodiment of the present invention, the step (S1B-4) may further comprise hydrolyzing the compound represented by Chemical Formula 22 in the presence of an acid to prepare the compound represented by Chemical Formula 3, but is not limited thereto.

In yet another embodiment of the present invention, the metal catalyst may be one or more selected from the group consisting of platinum black, rhodium, palladium carbon, and Raney-nickel (Raney-Ni), but is not limited thereto.

In yet another embodiment of the present invention, a compound represented by the following Chemical Formula 23 is prepared when the compound represented by Chemical Formula 9 is allowed to react with the compound represented by Chemical Formula 11 in the presence of a base in the step (S1B), but is not limited thereto.

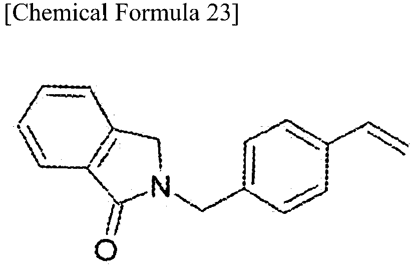

In yet another embodiment of the present invention, the step (S1B) may further comprise the following steps, but is not limited thereto:
(S1Ba-1) allowing the compound represented by Chemical Formula 23 to react with a peroxide to prepare a compound represented by the following Chemical Formula 24; and
(S1Ba-2) allowing the compound represented by Chemical Formula 24 to react with a compound represented by the following Chemical Formula 25 in the presence of a base to prepare the compound represented by Chemical Formula 4; or
(S1Bb-1) phosphorylating the compound represented by Chemical Formula 23 in the presence of a base to prepare a compound represented by the following Chemical Formula 26; and
(S1Bb-2) allowing the compound represented by Chemical Formula 26 to react with a compound represented by the following Chemical Formula 27 in the presence of a base to prepare the compound represented by Chemical Formula 5.

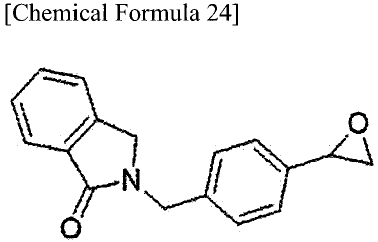

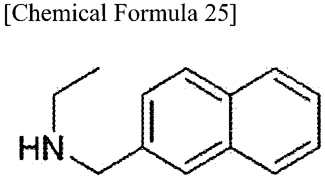

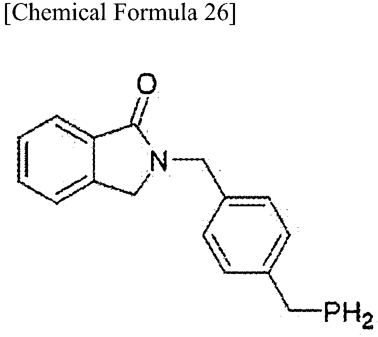

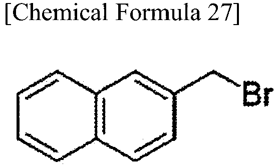

In yet another embodiment of the present invention, the peroxide may be one or more selected from the group consisting of meta-chloroperoxybenzoic acid (m-CPBA), H₂O₂, dimethyldioxirane (DMDO), and oxone, but is not limited thereto.

In yet another embodiment of the present invention, the compound represented by Chemical Formula 25 may be produced by allowing a compound represented by the following Chemical Formula 28 to react with ethylamine in the presence of a reducing agent, but is not limited thereto.

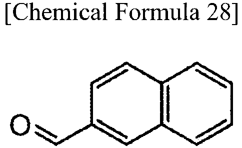

In yet another embodiment of the present invention, the reducing agent may be one or more selected from the group consisting of sodium cyanoborohydride (NaBH₃CN), sodium triacetoxyborohydride (NaBH(OAc)₃), and sodium borohydride (NaBH₄), but is not limited thereto.

In yet another embodiment of the present invention, the acid may be one or more selected from the group consisting of pivalic acid (PivOH), acetic acid (AcOH), trifluoromethanesulfonic acid (TfOH), paratoluenesulfonic acid (TsOH), benzoic acid, zinc bromide (ZnBr₂), hydrogen chloride (HCl), and trifluoroacetic acid (TFA), but is not limited thereto.

In yet another embodiment of the present invention, the base may be one or more selected from the group consisting of potassium carbonate (K₂CO₃), sodium carbonate (Na₂CO₃), sodium bicarbonate (NaHCO₃), potassium bicarbonate (KHCO₃), cupric bromide (CuBr₂), cesium carbonate (Cs₂CO₃), lithium hydroxide (LiOH), sodium hydride (NaH), potassium hydride (KH), potassium hydroxide (KOH), triethylamine (TEA), N,N-diisopropylethylamine (DIPEA), pyridine, and piperidine, but is not limited thereto.

In yet another embodiment of the present invention, the method may be carried out in one or more solvents selected from the group consisting of an organic solvent, water, and a mixture thereof, but is not limited thereto.

In yet another embodiment of the present invention, the organic solvent may be one or more selected from the group consisting of dichloromethane (DCM), dichloroethane (DCE), 1,4-dioxane, tetrahydrofuran (THF), toluene, hexane, benzene, xylene, chlorobenzene, methanol (MeOH), ethanol (EtOH), t-amyl alcohol (t-AmOH), trifluoroethanol (TFE), hexafluoroisopropanol (HFIP), acetonitrile (ACN), dimethylformamide (DMF), nitromethane, trimethoxymethane (CH(OMe)₃), acetic acid, isopropanol (IPA), and chloroform, but is not limited thereto.

Additionally, the present invention provides a pharmaceutical composition for the prevention or treatment of brain tumors, comprising, as an active ingredient, at least one compound selected from the group consisting of the compounds represented by Chemical Formulae 1 to 6 or pharmaceutically acceptable salts thereof.

Furthermore, the present invention provides a method for preventing or treating brain tumors, comprising administering, to a subject in need thereof, at least one compound selected from the group consisting of the compounds represented by Chemical Formulae 1 to 6 or pharmaceutically acceptable salts thereof, or a composition comprising the same as an active ingredient.

Furthermore, the present invention provides the use of at least one compound selected from the group consisting of the compounds represented by Chemical Formulae 1 to 6 or pharmaceutically acceptable salts thereof, or a composition comprising the same as an active ingredient, for the prevention or treatment of brain tumors.

Furthermore, the present invention provides the use of at least one compound selected from the group consisting of the compounds represented by Chemical Formulae 1 to 6 for the manufacture of a medicament for treating brain tumors.

In one embodiment of the present invention, the brain tumors may be associated with at least one mutation selected from the group consisting of EGFR (epidermal growth factor receptor) and ANO1 (Anoctamin 1), but is not limited thereto.

In another embodiment of the present invention, the compound or pharmaceutically acceptable salts thereof may simultaneously inhibit EGFR and ANO1 (for example, by suppressing expression and/or activity), but is not limited thereto. In other words, the compound or pharmaceutically acceptable salts thereof may be a dual inhibitor targeting both EGFR and ANO1.

In yet another embodiment of the present invention, the compound or pharmaceutically acceptable salts thereof may satisfy at least one property selected from the group consisting of:
(a) inhibiting tumor metastasis; and
(b) suppressing resistance of cancer to an anticancer agent, but is not limited thereto.

In yet another embodiment of the present invention, the anticancer agent may be a targeted anticancer agent against tyrosine kinase, but is not limited thereto.

In yet another embodiment of the present invention, the tyrosine kinase may be one or more selected from the group consisting of EGFR (epidermal growth factor receptor), ALK (anaplastic lymphoma kinase), ROS1 (ROS proto-oncogene 1), BRAF (B-Raf proto-oncogene), HER2 (human epidermal growth factor receptor 2), RET (Ret proto-oncogene), NTRK1 (neurotrophic receptor tyrosine kinase 1), MET (mesenchymal-epithelial transition factor), and NRG1 (neuregulin 1), but is not limited thereto.

In yet another embodiment of the present invention, the pharmaceutical composition may be administered in combination with a targeted anticancer agent against tyrosine kinase, but is not limited thereto.

In yet another embodiment of the present invention, the pharmaceutical composition may be administered simultaneously, separately, or sequentially with the targeted anticancer agent, but is not limited thereto.

Additionally, the present invention provides a pharmaceutical composition for the prevention or treatment of brain tumors, comprising as active ingredients (i) at least one compound selected from the group consisting of the compounds represented by Chemical Formulae 1 to 6 or pharmaceutically acceptable salts thereof, and (ii) a targeted anticancer agent against EGFR.

Furthermore, the present invention provides a method for preventing or treating brain tumors, comprising administering, to a subject in need thereof, (i) at least one compound selected from the group consisting of the compounds represented by Chemical Formulae 1 to 6 or pharmaceutically acceptable salts thereof, and (ii) a targeted anticancer agent against EGFR.

Furthermore, the present invention provides the use of a composition comprising as active ingredients (i) at least one compound selected from the group consisting of the compounds represented by Chemical Formulae 1 to 6 or pharmaceutically acceptable salts thereof, and (ii) a targeted anticancer agent against EGFR, for the prevention or treatment of brain tumors.

Furthermore, the present invention provides the use of a composition comprising as active ingredients (i) at least one compound selected from the group consisting of the compounds represented by Chemical Formulae 1 to 6 or pharmaceutically acceptable salts thereof, and (ii) a targeted anticancer agent against EGFR, for the manufacture of a medicament for treating brain tumors.

In one embodiment of the present invention, the compound or pharmaceutically acceptable salts thereof may inhibit resistance of brain tumor cells to the targeted anticancer agent against EGFR, but is not limited thereto.

In another embodiment of the present invention, the composition may be in the form of a mixture formulation in which the compound or pharmaceutically acceptable salts thereof and the targeted anticancer agent against EGFR are mixed together, but is not limited thereto.

In yet another embodiment of the present invention, the composition may be in a form in which the compound or pharmaceutically acceptable salts thereof and the targeted anticancer agent against EGFR are separately formulated and administered simultaneously, separately, or sequentially, but is not limited thereto.

In addition, the present invention provides a pharmaceutical composition for enhancing the anticancer effect of a brain tumor anticancer agent, comprising, as an active ingredient, at least one compound selected from the group consisting of the compounds represented by Chemical Formulae 1 to 6 or pharmaceutically acceptable salts thereof.

Furthermore, the present invention provides a method for enhancing the anticancer effect of an anticancer agent for brain tumors, comprising administering, to a subject in need thereof, at least one compound selected from the group consisting of the compounds represented by Chemical Formulae 1 to 6 or pharmaceutically acceptable salts thereof, or a composition comprising the same as an active ingredient.

Furthermore, the present invention provides the use of at least one compound selected from the group consisting of the compounds represented by Chemical Formulae 1 to 6 or pharmaceutically acceptable salts thereof, or a composition comprising the same as an active ingredient, for enhancing the anticancer effect of an anticancer agent for brain tumors.

Furthermore, the present invention provides the use of at least one compound selected from the group consisting of the compounds represented by Chemical Formulae 1 to 6 or pharmaceutically acceptable salts thereof for the manufacture of a medicament for enhancing the anticancer effect of an anticancer agent for brain tumors.

Furthermore, the present invention provides the use of one or more compounds selected from the group consisting of the compounds represented by Chemical Formulae 1 to 6 or pharmaceutically acceptable salts thereof for the manufacture of an inhibitor of resistance of brain tumor cells to a brain tumor anticancer agent.

In one embodiment of the present invention, the brain tumor anticancer agent may be a targeted anticancer agent against tyrosine kinase, but is not limited thereto.

In another embodiment of the present invention, the tyrosine kinase may be one or more selected from the group consisting of EGFR (epidermal growth factor receptor), ALK (anaplastic lymphoma kinase), ROS1 (ROS proto-oncogene 1), BRAF (B-Raf proto-oncogene), HER2 (human epidermal growth factor receptor 2), RET (Ret proto-oncogene), NTRK1 (neurotrophic receptor tyrosine kinase 1), MET (mesenchymal-epithelial transition factor), and NRG1 (neuregulin 1), but is not limited thereto.

In yet another embodiment of the present invention, the composition may be administered simultaneously, separately, or sequentially with the brain tumor anticancer agent, but is not limited thereto.

### [Advantageous Effects]

The present invention relates to the use of a novel Anoctamin 1 (ANO1) inhibitory compound for the prevention and treatment of brain tumor, which has been completed by confirming that the compound is capable of suppressing brain tumor by inhibiting the expression or activity of ANO1, a calcium-dependent chloride channel. Brain tumor cells treated with the compound exhibited significantly inhibited growth, migration, and proliferation, and such inhibitory effects were found to be superior to those of conventional ANO1 inhibitors. In particular, the compound of the present invention is capable of simultaneously inhibiting ANO1 and EGFR in brain tumor cells, and may not only exhibit a more potent anticancer effect through the dual inhibition of these two proteins, but also enhance the efficacy of anticancer agents, such as EGFR-targeted anticancer drugs, and suppress drug resistance when used in combination therewith. Therefore, the compound may be used as a dual-target anticancer agent against ANO1 and EGFR by itself, and may also be utilized as a combination agent for EGFR-targeted therapy, and thus is expected to be useful in various applications in the prevention and treatment of brain tumor.

### [Description of Drawings]

FIG. 1 shows the names, structural formulas, and basic information of the compounds AON-MG23-01 and AON-MG23-02 according to the present invention.
FIG. 2 shows the results of a CCK assay performed after treating human glioblastoma cell lines with various concentrations of the compounds AON-MG23-01 or AON-MG23-02 of the present invention, in order to confirm the inhibitory effect of the compounds AON-MG23-01 and AON-MG23-02 on cell growth in human glioblastoma cells.
FIG. 3 shows the results obtained by photographing and analyzing the extent of cell migration over time after treating human glioblastoma cell lines with various concentrations of the compounds AON-MG23-01 or AON-MG23-02 of the present invention, in order to confirm the inhibitory effect of the compounds AON-MG23-01 and AON-MG23-02 of the present invention on cell migration in human glioblastoma cells.
FIG. 4 shows the results obtained by photographing and analyzing the extent of Matrigel invasion of human glioblastoma cell lines cultured on Matrigel after treating the cells with various concentrations of the compounds AON-MG23-01 or AON-MG23-02 of the present invention, in order to confirm the inhibitory effects of the compounds AON-MG23-01 and AON-MG23-02 of the present invention on glioblastoma invasion.
FIG. 5 shows the results of a western blot analysis performed after treating human glioblastoma cell lines with various concentrations of the compounds AON-MG23-01 or AON-MG23-02 of the present invention, in order to confirm the effects of the compounds AON-MG23-01 and AON-MG23-02 on the expression of EGFR and ANO1 proteins in glioblastoma. The left panel shows the images of protein bands detected by western blot, and the right panel shows the quantification of EGFR and ANO1 protein levels.
FIGS. 6A and 6B show the results of whole-cell patch clamp experiments performed after treating human glioblastoma cell lines with the compounds AON-MG23-01 or AON-MG23-02 of the present invention, in order to confirm whether the compounds inhibit ANO1 activity in glioblastoma. FIG. 6A shows the results of measuring calcium-dependent chloride ion channel currents from -100 mV to +100 mV, and FIG. 6B shows the current-voltage (I/V) plot at +80 mV derived from FIG. 6A.
FIGS. 7A and 7B show the results comparing the inhibitory effects of the compound AON-MG23-02 of the present invention, an EGFR inhibitor, and an ANO1 inhibitor on the invasion of human glioblastoma.
FIGS. 8A and 8B show the results comparing the inhibitory effects of the compound AON-MG23-02 of the present invention, an EGFR inhibitor, and an ANO1 inhibitor on the expression of ANO1, EGFR, and pEGFR proteins in glioblastoma cell lines.
FIGS. 9A to 9D show the results of western blot analyses performed after treating human glioblastoma cell lines with various concentrations of the derivatives of the compound AON-MG23-02 of the present invention, namely AON-MG23-05, AON-MG23-06, AON-MG23-07, or AON-MG23-08, to examine the effects thereof on the expression of EGFR and ANO1 proteins in glioblastoma. Specifically, FIG. 9A shows the results for AON-MG23-05, FIG. 9B for AON-MG23-06, FIG. 9C for AON-MG23-07, and FIG. 9D for AON-MG23-08, confirming the expression levels of ANO1 and EGFR proteins by western blot analysis.
FIGS. 10A and 10B show the results obtained by photographing and analyzing the extent of Matrigel invasion of human glioblastoma cell lines cultured on Matrigel after treating the cells with various concentrations of the derivatives of the compound AON-MG23-02 of the present invention, namely AON-MG23-05, AON-MG23-06, AON-MG23-07, or AON-MG23-08, in order to confirm the inhibitory effects of these derivatives on glioblastoma invasion.
FIG. 11 shows the H NMR data of AON-MG23-05, a derivative of the compound AON-MG23-02 of the present invention.
FIG. 12 shows the H NMR data of AON-MG23-06, a derivative of the compound AON-MG23-02 of the present invention.
FIG. 13 shows the H NMR data of AON-MG23-07, a derivative of the compound AON-MG23-02 of the present invention.
FIG. 14 shows the H NMR data of AON-MG23-08, a derivative of the compound AON-MG23-02 of the present invention.

### [Best Mode]

The present invention relates to novel compounds having an inhibitory effect on Anoctamin 1 (ANO1), and was completed by confirming that the compounds can inhibit the expression or activity of ANO1, a calcium-dependent chloride channel, thereby suppressing the growth and metastasis of brain tumor cells. In particular, the compounds of the present invention have been confirmed to effectively inhibit the expression of EGFR together with ANO1 in brain tumor cells, and are therefore expected to exhibit stronger anticancer effects than conventional single inhibitors of ANO1 or EGFR, and further to suppress the development of resistance of brain tumor cells to EGFR-targeted therapeutics.

Accordingly, an object of the present invention is to provide one or more compounds selected from the group consisting of the compounds represented by Chemical Formulae 1 to 6 or pharmaceutically acceptable salts thereof.

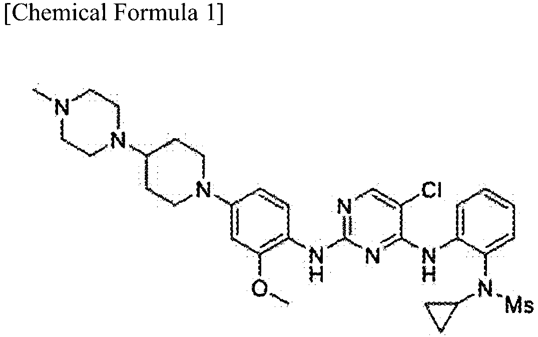

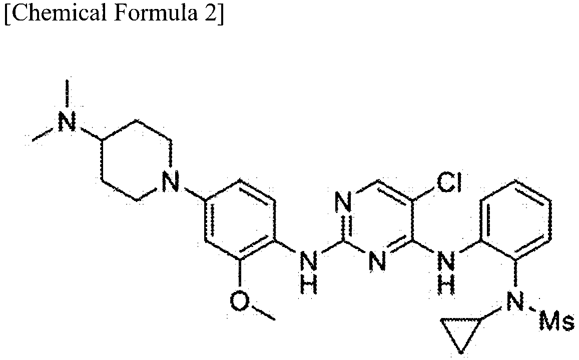

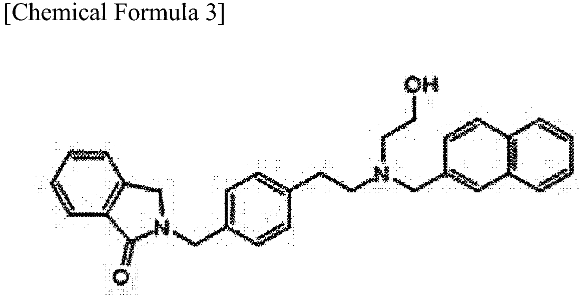

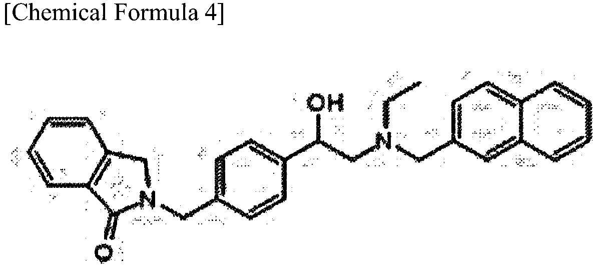

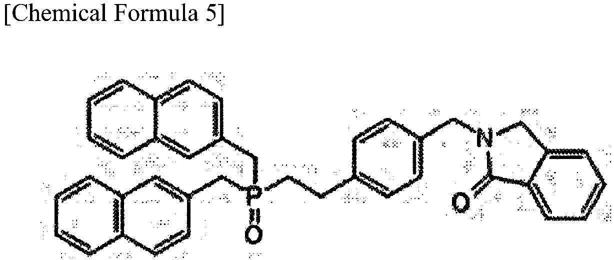

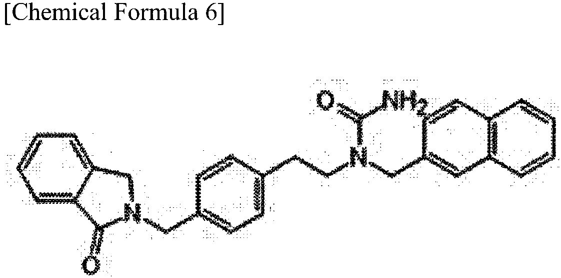

In the present invention, the compound represented by Chemical Formula 1 may be referred to as "N-(2-((5-chloro-2-((2-methoxy-4-(4-(4-methylpiperazin-1-yl)piperidin-1-yl)phenyl)amino)pyrimidin-4-yl)amino)phenyl)-N-cyclopropylmethanesulfonamide" or "AON-MG23-01." In addition, the compound represented by Chemical Formula 2 may be referred to as "N-(2-((5-chloro-2-((4-(4-(dimethylamino)piperidin-1-yl)-2-methoxyphenyl)amino)pyrimidin-4-yl)amino)phenyl)-N-cyclopropylmethanesulfonamide" or "AON-MG23-02."

As used herein, the term "Ms" refers to methylsulfonyl, that is, -SO₂(CH₃).

In addition, the compound represented by Chemical Formula 3 may be referred to as "2-(4-(2-((2-hydroxyethyl)(naphthalen-2-ylmethyl)amino)ethyl)benzyl)isoindolin-1-one," "AON-MG23-05," or "Target A."

The compound represented by Chemical Formula 4 may be referred to as "2-(4-(2-(ethyl(naphthalen-2-ylmethyl)amino)-1-hydroxyethyl)benzyl)isoindolin-1-one," "AON-MG23-06," or "Target B."

The compound represented by Chemical Formula 5 may be referred to as "2-(4-(2-(bis(naphthalen-2-ylmethyl)phosphoryl)ethyl)benzyl)isoindolin-1-one," "AON-MG23-07," or "Target C."

The compound represented by Chemical Formula 6 may be referred to as "1-(naphthalen-2-ylmethyl)-1-(4-((1-oxoisoindolin-2-yl)methyl)phenethyl)urea," "AON-MG23-08," or "Target D."

Unless otherwise specified herein, the terms "compound of the present invention" or "compound represented by Chemical Formulae 1 to 28," and the like, are intended to include the compounds represented by Chemical Formulae 1 to 28 themselves, salts thereof, and isomers thereof.

In the present invention, the term "pharmaceutically acceptable salt" includes a salt derived from a pharmaceutically acceptable inorganic acid, organic acid, or base.

As used herein, the term "pharmaceutically acceptable" refers to a compound or composition that is suitable for contact with the tissues of a subject (for example, a human) without excessive toxicity, irritation, allergic response, or other problems or complications, and that has a reasonable benefit-to-risk ratio within the scope of sound medical judgment.

Examples of suitable acids include hydrochloric acid, hydrobromic acid, sulfuric acid, nitric acid, perchloric acid, hydroiodic acid, fumaric acid, maleic acid, phosphoric acid, glycolic acid, lactic acid, salicylic acid, succinic acid, p-toluenesulfonic acid, tartaric acid, (+)-L-tartaric acid, di-L-tartaric acid, acetic acid, trichloroacetic acid or trifluoroacetic acid, 2,2-dichloroacetic acid, acylated amino acids, adipic acid, alginic acid, ascorbic acid, L-aspartic acid, 4-acetamidobenzoic acid, (+)-camphoric acid, camphorsulfonic acid, (+)-(1S)-camphorsulfonic acid, capric acid, caproic acid, caprylic acid, cinnamic acid, cyclamic acid, dodecylsulfuric acid, ethan-1,2-disulfonic acid, ethanesulfonic acid, 2-hydroxyethanesulfonic acid, galactaric acid, gentisic acid, glucoheptanoic acid, D-gluconic acid, D-glucuronic acid, L-glutamic acid, α-oxoglutaric acid, hippuric acid, (+)-L-lactic acid, (±)-DL-lactic acid, lactobionic acid, (-)-L-malic acid, (±)-DL-mandelic acid, citric acid, methanesulfonic acid, formic acid, benzoic acid, malonic acid, gluconic acid, naphthalene-2-sulfonic acid, naphthalene-1,5-disulfonic acid, benzenesulfonic acid, 1-hydroxy-2-naphthoic acid, nicotinic acid, oleic acid, orotic acid, oxalic acid, di-oxalic acid, palmitic acid, palmityl acid, L-pyroglutamic acid, salicylic acid, 4-amino-salicylic acid, sebacic acid, stearic acid, tannic acid, thiocyanic acid, camphosic acid, and undecylic acid. Acid addition salts may be prepared by conventional methods, for example, by dissolving the compound in an aqueous solution of an excess acid and precipitating the salt using a water-miscible organic solvent such as methanol, ethanol, acetone, or acetonitrile. Alternatively, the salt may be prepared by heating equimolar amounts of the compound and an acid or alcohol in water, followed by evaporating and drying the mixture, or by suction-filtering the precipitated salt.

The scope of the compounds of the present invention may include not only pharmaceutically acceptable salts but also all isomers, hydrates, and solvates that can be prepared by conventional methods.

In the present invention, the term "isomer" refers to a compound having the same molecular formula but differing in the connectivity or spatial arrangement of constituent atoms within the molecule. The isomers may include, for example, structural isomers and stereoisomers. The stereoisomers may be diastereomers or enantiomers. An enantiomer refers to an isomer that is non-superimposable on its mirror image, similar to the relationship between the left and right hands, and is also referred to as an optical isomer. Enantiomers are classified as R (Rectus; clockwise) or S (Sinister; counterclockwise) when the chiral carbon atom possesses four different substituents. Diastereomers refer to stereoisomers that are not mirror images of each other, and they may include cis-trans isomers arising from differences in the spatial arrangement of atoms.

In addition, the present invention provides a method for preparing the compounds represented by Chemical Formulae 1 to 6, comprising: (S1A) preparing a compound represented by the following Chemical Formula 8 by allowing a compound represented by the following Chemical Formula 7 to react with methanesulfonyl chloride (MsCl) and cyclopropylamine in the presence of a base (Reaction Scheme 2); or
(S1B) allowing a compound represented by the following Chemical Formula 9 to react with a compound represented by the following Chemical Formula 10 or 11 in the presence of a base (Reaction Scheme 7 or 13):

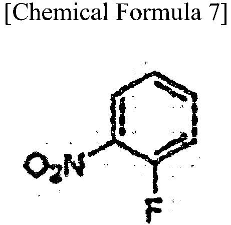

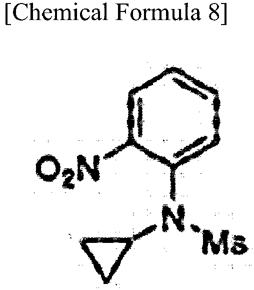

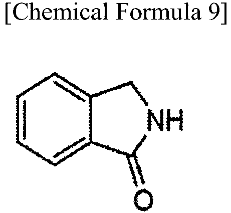

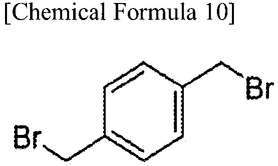

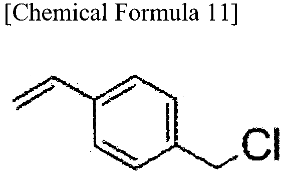

According to one embodiment of the present invention, the base used in the step (S1A) or (S1B) may be Cs₂CO₃, but is not limited thereto.

According to one embodiment of the present invention, the step (S1A) may further comprise the following steps, but is not limited thereto:
(S1A-1) reducing the compound (the nitro group) represented by Chemical Formula 8 with zinc in the presence of ammonium chloride to prepare a compound represented by Chemical Formula 12 (Reaction Scheme 3);
(S1A-2) allowing the compound represented by Chemical Formula 12 to react with 2,4,5-trichloropyrimidine in the presence of a base to prepare a compound represented by Chemical Formula 13 (Reaction Scheme 4); and
(S1A-3) allowing the compound represented by Chemical Formula 13 to react with a compound represented by Chemical Formula 14 or Chemical Formula 15 in the presence of an acid to prepare a compound represented by Chemical Formula 1 or Chemical Formula 2 (Reaction Scheme 5 or 6).

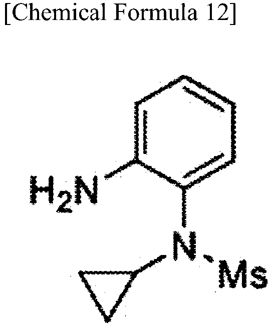

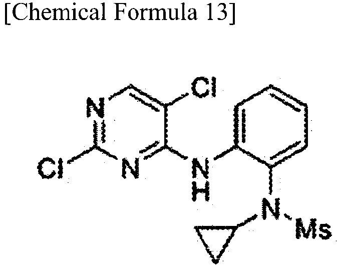

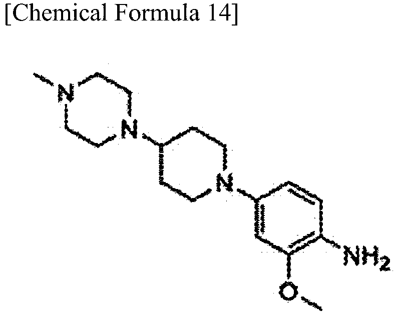

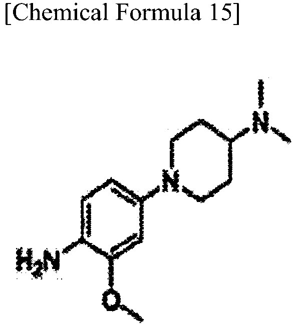

According to one embodiment of the present invention, when, in the step (S1A-3), the compound represented by Chemical Formula 13 is allowed to react with the compound represented by Chemical Formula 14 in the presence of an acid, a compound represented by Chemical Formula 1 is prepared (Reaction Scheme 5),
and when the compound represented by Chemical Formula 13 is allowed to react with the compound represented by Chemical Formula 15 in the presence of an acid, a compound represented by Chemical Formula 2 may be prepared (Reaction Scheme 6), and, specifically, the step (S1A-3) may be the reaction of the compound represented by Chemical Formula 13 with the amino group of the compound represented by Chemical Formula 14 or Chemical Formula 15.

According to one embodiment of the present invention, in the step (S1A-2), the base is DIPEA (N,N-diisopropylethylamine), and in the step (S1A-3), the acid may be TFA (trifluoroacetic acid), but is not limited thereto.

In the present invention, when, in the step (S1B), the compound represented by Chemical Formula 9 reacts with the compound represented by Chemical Formula 10 in the presence of a base, a compound represented by the following Chemical Formula 16 may be produced (Reaction Scheme 7 or 19), but is not limited thereto, and according to one embodiment of the present invention, the base may be Cs₂CO₃, but is not limited thereto.

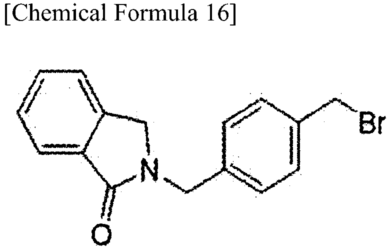

According to one embodiment of the present invention, the step (S1B) may further comprise the following steps, but is not limited thereto:
(S1B-1) allowing the compound represented by Chemical Formula 16 to react with trimethylsilyl cyanide in the presence of a base to prepare a compound represented by the following Chemical Formula 17 (Reaction Scheme 8 or 20);
(S1B-2) hydrogenating the compound represented by Chemical Formula 17 in the presence of a metal catalyst to prepare a compound represented by the following Chemical Formula 18 (Reaction Scheme 9 or 21);
(S1B-3) allowing the compound represented by Chemical Formula 18 to react with a compound represented by the following Chemical Formula 19 in the presence of a reducing agent to prepare a compound represented by the following Chemical Formula 20 (Reaction Scheme 10 or 22); and
(S1B-4) allowing the compound represented by Chemical Formula 20 to react with NCO- to prepare the compound represented by Chemical Formula 6 (Reaction Scheme 23); or allowing the compound represented by Chemical Formula 20 to react with a compound represented by the following Chemical Formula 21 in the presence of a base to prepare a compound represented by the following Chemical Formula 22 (Reaction Scheme 11), and according to one embodiment of the present invention, the base in the step (S1B-1) is Cs₂CO₃ and the base in the step (S1B-4) may be K₂CO₃, but is not limited thereto.

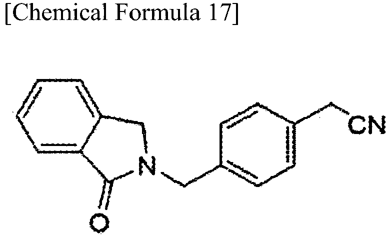

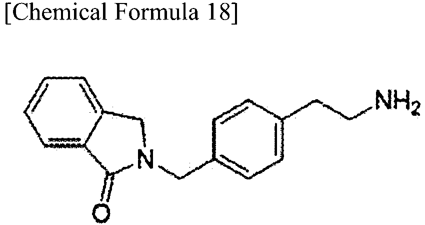

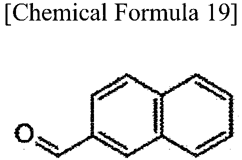

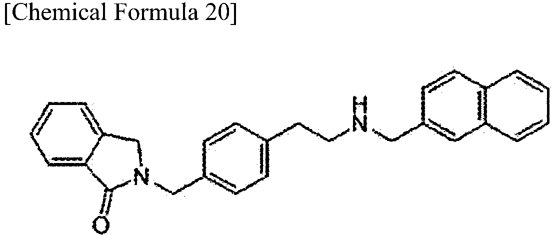

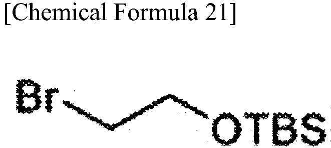

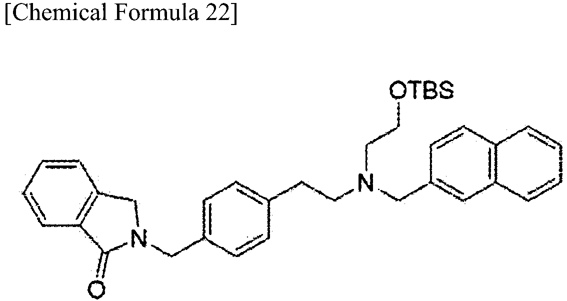

In the present invention, the step (S1B-4) may further comprise hydrolyzing the compound represented by Chemical Formula 22 in the presence of an acid to prepare the compound represented by Chemical Formula 3 (Reaction Scheme 12), but is not limited thereto, and according to one embodiment of the present invention, the acid used in the step (S1B-4) may be hydrogen chloride (HCl), but is not limited thereto.

In the present invention, the metal catalyst may be one or more selected from the group consisting of platinum black, rhodium, palladium carbon, and Raney-nickel (Raney-Ni), and according to one embodiment of the present invention, the metal catalyst may be Raney-nickel (Raney-Ni), but is not limited thereto.

In the present invention, the reducing agent may be one or more selected from the group consisting of sodium cyanoborohydride (NaBH₃CN), sodium triacetoxyborohydride (NaBH(OAc)₃), and sodium borohydride (NaBH₄), and according to one embodiment of the present invention, the reducing agent used in the step (S1B-3) may be sodium cyanoborohydride (NaBH₃CN), but is not limited thereto.

In the present invention, when, in the step (S1B), the compound represented by Chemical Formula 9 reacts with the compound represented by Chemical Formula 11 in the presence of a base, a compound represented by the following Chemical Formula 23 may be produced (Reaction Scheme 13), but is not limited thereto.

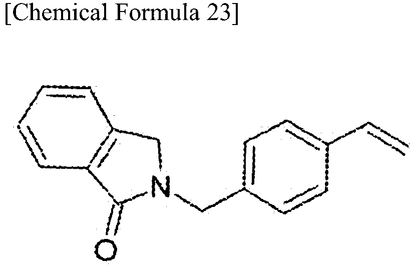

In the present invention, the step (S1B) may further comprise the following steps, but is not limited thereto:
(S1Ba-1) allowing the compound represented by Chemical Formula 23 to react with a peroxide to prepare a compound represented by the following Chemical Formula 24 (Reaction Scheme 14); and
(S1Ba-2) allowing the compound represented by Chemical Formula 24 to react with a compound represented by the following Chemical Formula 25 in the presence of a base to prepare the compound represented by Chemical Formula 4 (Reaction Scheme 16); or
(S1Bb-1) phosphorylating the compound represented by Chemical Formula 23 in the presence of a base to prepare a compound represented by the following Chemical Formula 26, specifically preparing the compound represented by Chemical Formula 26 through a β-alkylstyrene phosphorylation reaction (Reaction Scheme 17); and
(S 1Bb-2) allowing the compound represented by Chemical Formula 26 to react with a compound represented by the following Chemical Formula 27 in the presence of a base to prepare the compound represented by Chemical Formula 5 (Reaction Scheme 18).

According to one embodiment of the present invention, the base in the step (S1Ba-2) is DIEA (N,N-diisopropylethylamine), the base in the step (S1Bb-1) is KOH, and the base in the step (S1Bb-2) may be NaH, but is not limited thereto.

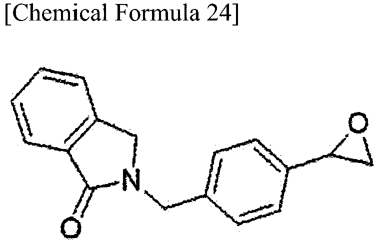

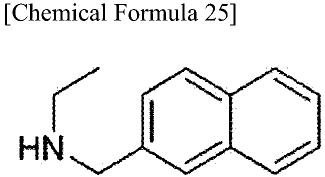

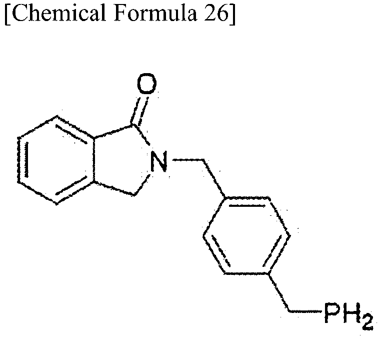

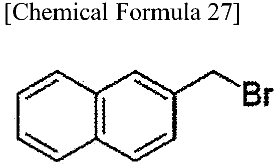

In the present invention, the compound represented by Chemical Formula 25 may be prepared by allowing the compound represented by the following Chemical Formula 28 to react with ethylamine in the presence of a reducing agent (Reaction Scheme 15), and according to one embodiment of the present invention, the reducing agent may be sodium triacetoxyborohydride (NaBH(OAc)₃), but is not limited thereto.

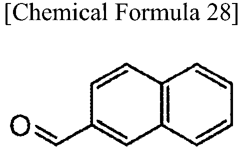

In the present invention, the peroxide may be one or more selected from the group consisting of m-CPBA (meta-chloroperoxybenzoic acid), hydrogen peroxide (H₂O₂), dimethyldioxirane (DMDO), and oxone, and according to one embodiment of the present invention, the peroxide may be m-CPBA (meta-chloroperoxybenzoic acid), but is not limited thereto.

In the present invention, the acid may be one or more selected from the group consisting of pivalic acid (PivOH), acetic acid (AcOH), trifluoromethanesulfonic acid (TfOH), p-toluenesulfonic acid (TsOH), benzoic acid, zinc bromide (ZnBr₂), hydrogen chloride (HCl), and trifluoroacetic acid (TFA), but is not limited thereto.

In the present invention, the base may be one or more selected from the group consisting of potassium carbonate (K₂CO₃), sodium carbonate (Na₂CO₃), sodium bicarbonate (NaHCO₃), potassium bicarbonate (KHCO₃), cupric bromide (CuBr₂), cesium carbonate (Cs₂CO₃), lithium hydroxide (LiOH), sodium hydride (NaH), potassium hydride (KH), potassium hydroxide (KOH), triethylamine (TEA), N,N-diisopropylethylamine (DIPEA), pyridine, and piperidine, but is not limited thereto.

In the present invention, the preparation method may be carried out in one or more solvents selected from the group consisting of organic solvents, water, and mixtures thereof, and the organic solvent may be one or more selected from the group consisting of dichloromethane (DCM), dichloroethane (DCE), 1,4-dioxane, tetrahydrofuran (THF), toluene, hexane, benzene, xylene, chlorobenzene, methanol (MeOH), ethanol (EtOH), t-amyl alcohol (t-AmOH), trifluoroethanol (TFE), hexafluoroisopropanol (HFIP), acetonitrile (ACN), dimethylformamide (DMF), nitromethane, trimethoxymethane (CH(OMe)₃), acetic acid (acetic anhydride, acetic acid, or a mixture of acetic anhydride and acetic acid), and chloroform, but is not limited thereto.

In addition, the present invention provides a method for preparing a compound represented by Chemical Formula 1, comprising the following steps:
(a) allowing the compound represented by Chemical Formula 7 to react with methanesulfonyl chloride (MsCl) and cyclopropylamine in the presence of a base to prepare a compound represented by Chemical Formula 8 (Reaction Scheme 2);
(b) reducing the compound (the nitro group) represented by Chemical Formula 8 with zinc in the presence of ammonium chloride to prepare a compound represented by Chemical Formula 12 (Reaction Scheme 3);
(c) allowing the compound represented by Chemical Formula 12 to react with 2,4,5-trichloropyrimidine in the presence of a base to prepare a compound represented by the following Chemical Formula 13 (Reaction Scheme 4); and
(d) allowing the compound represented by Chemical Formula 13 to react with the compound represented by the following Chemical Formula 14 in the presence of an acid to prepare the compound represented by Chemical Formula 1 (Reaction Scheme 5).

In addition, the present invention provides a method for preparing a compound represented by Chemical Formula 2, comprising the following steps:
(a) allowing the compound represented by Chemical Formula 7 to react with methanesulfonyl chloride (MsCl) and cyclopropylamine in the presence of a base to prepare a compound represented by Chemical Formula 8 (Reaction Scheme 2);
(b) reducing the compound (the nitro group) represented by Chemical Formula 8 with zinc in the presence of ammonium chloride to prepare a compound represented by Chemical Formula 12 (Reaction Scheme 3);
(c) allowing the compound represented by Chemical Formula 12 to react with 2,4,5-trichloropyrimidine in the presence of a base to prepare a compound represented by the following Chemical Formula 13 (Reaction Scheme 4); and
(d) allowing the compound represented by Chemical Formula 13 to react with the compound represented by the following Chemical Formula 15 in the presence of an acid to prepare the compound represented by Chemical Formula 2 (Reaction Scheme 6).

In addition, the present invention provides a method for preparing a compound represented by Chemical Formula 3, comprising the following steps:
(a) allowing the compound represented by Chemical Formula 9 to react with the compound represented by Chemical Formula 10 in the presence of a base to prepare a compound represented by Chemical Formula 16 (Reaction Scheme 7);
(b) allowing the compound represented by Chemical Formula 16 to react with trimethylsilyl cyanide in the presence of a base to prepare a compound represented by Chemical Formula 17 (Reaction Scheme 8);
(c) hydrogenating the compound represented by Chemical Formula 17 in the presence of a metal catalyst to prepare a compound represented by Chemical Formula 18 (Reaction Scheme 9);
(d) allowing the compound represented by Chemical Formula 18 to react with the compound represented by Chemical Formula 19 in the presence of a reducing agent to prepare a compound represented by Chemical Formula 20 (Reaction Scheme 10);
(d) allowing the compound represented by Chemical Formula 20 to react with the compound represented by Chemical Formula 21 in the presence of a base to prepare a compound represented by Chemical Formula 22 (Reaction Scheme 11); and
(e) hydrolyzing the compound represented by Chemical Formula 22 in the presence of an acid to prepare the compound represented by Chemical Formula 3 (Reaction Scheme 12).

In addition, the present invention provides a method for preparing a compound represented by Chemical Formula 4, comprising the following steps:
(a) allowing the compound represented by Chemical Formula 9 to react with the compound represented by Chemical Formula 11 in the presence of a base to prepare a compound represented by Chemical Formula 23 (Reaction Scheme 13);
(b) allowing the compound represented by Chemical Formula 23 to react with a peroxide to prepare a compound represented by Chemical Formula 24 (Reaction Scheme 14); and
(c) allowing the compound represented by Chemical Formula 24 to react with the compound represented by Chemical Formula 25 in the presence of a base to prepare the compound represented by Chemical Formula 4 (Reaction Scheme 16).

In addition, the present invention provides a method for preparing a compound represented by Chemical Formula 5, comprising the following steps:
(a) allowing the compound represented by Chemical Formula 9 to react with the compound represented by Chemical Formula 11 in the presence of a base to prepare a compound represented by Chemical Formula 23 (Reaction Scheme 13);
(b) phosphorylating the compound represented by Chemical Formula 23 in the presence of a base to prepare a compound represented by Chemical Formula 26, specifically a step of preparing the compound represented by Chemical Formula 26 through a β-alkylstyrene phosphorylation reaction (Reaction Scheme 17); and
(c) allowing the compound represented by Chemical Formula 26 to react with the compound represented by Chemical Formula 27 in the presence of a base to prepare the compound represented by Chemical Formula 5 (Reaction Scheme 18).

In addition, the present invention provides a method for preparing a compound represented by Chemical Formula 6, comprising the following steps:
(a) allowing the compound represented by Chemical Formula 9 to react with the compound represented by Chemical Formula 10 in the presence of a base to prepare a compound represented by Chemical Formula 16 (Reaction Scheme 19);
(b) allowing the compound represented by Chemical Formula 16 to react with trimethylsilyl cyanide in the presence of a base to prepare a compound represented by Chemical Formula 17 (Reaction Scheme 20);
(c) hydrogenating the compound represented by Chemical Formula 17 in the presence of a metal catalyst to prepare a compound represented by Chemical Formula 18 (Reaction Scheme 21);
(d) allowing the compound represented by Chemical Formula 18 to react with the compound represented by Chemical Formula 19 in the presence of a reducing agent to prepare a compound represented by Chemical Formula 20 (Reaction Scheme 22);
(d) allowing the compound represented by Chemical Formula 20 to react with NCO⁻to prepare the compound represented by Chemical Formula 6 (Reaction Scheme 23).

In addition, another object of the present invention is to provide a pharmaceutical composition for preventing or treating brain tumors, comprising, as an active ingredient, one or more compounds selected from the group consisting of the compounds represented by Chemical Formulae 1 to 6 or pharmaceutically acceptable salts thereof.

The compound according to the present invention may be used as an inhibitor of Anoctamin 1 (ANO1), a calcium-dependent chloride channel, for the prevention, improvement, and/or treatment of brain tumors. In particular, the compound according to the present invention is characterized in that it exhibits excellent anticancer effects by simultaneously inhibiting the expression or activity of EGFR and ANO1. The prevention and/or therapeutic effect of the brain tumor includes not only the effect of inhibiting the growth of brain tumor cells, but also the effect of suppressing the progression or aggravation of brain tumors caused by migration, invasion, and metastasis.

In the present invention, "EGFR (epidermal growth factor receptor)" is a transmembrane glycoprotein belonging to the protein kinase superfamily and serves as a receptor for the proteins of the epidermal growth factor (EGF) family. EGFR is a key regulator of cell growth, and when its ligand, epidermal growth factor (EGF), binds to EGFR, the receptor undergoes dimerization and tyrosine autophosphorylation, thereby inducing cell proliferation. Overexpression (amplification) or hyperactivation of EGFR is frequently observed in various cancers such as colorectal cancer, head and neck cancer, and glioblastoma, and somatic mutations associated with EGFR lead to continuous activation of EGFR, resulting in uncontrolled cell division. In particular, EGFR gene rearrangements and EGFR gene amplifications are patterns commonly observed in various cancers. Among them, the most common extracellular domain mutation is EGFRvIII. The EGFRvIII mutation is characterized by a deletion of approximately 801 base pairs corresponding to exons 2 through 7 of the EGFR gene, and the EGFR protein expressed therefrom lacks 267 amino acids in the extracellular domain. Unlike normal EGFR, which requires ligand binding for activation, the mutant EGFR remains constitutively active (PCT gain-of-function mutation). The EGFRvIII mutation is particularly prevalent in glioblastoma.

In the present invention, "Anoctamin 1 (ANO1, also referred to as transmembrane protein 16A (TMEM16A))" is a calcium-activated chloride ion channel (Cl⁻ channel). ANO1 is expressed in various normal cells including epithelial cells, smooth muscle cells, vascular endothelial cells, and neurons, and performs various physiological functions by regulating fluid secretion, muscle contraction, and nociception. However, ANO1 is also known to be associated with tumor invasiveness and poor prognosis in brain tumors, including glioblastoma. In fact, overexpression of ANO1 promotes signaling pathways that stimulate proliferation and migration in cancer cells, whereas inhibition of ANO1 has been reported to suppress cell migration and growth. Overexpression of ANO1 also appears to be associated with overexpression of EGFR and STAT3, and inhibition of ANO1 has been reported to enhance the response of head and neck squamous cell carcinoma to EGFR/HER2-targeted therapy. Accordingly, ANO1 has attracted attention as a target for cancer therapy; however, an ANO1 inhibitor exhibiting excellent anticancer efficacy has not yet been developed. The compound according to the present invention effectively suppresses the expression of ANO1 in brain tumor cells and has been confirmed to inhibit migration, proliferation, and metastasis of brain tumor cells more strongly than conventional ANO1 inhibitors, and therefore may be used for the prevention, treatment, and inhibition of metastasis of brain tumors. Furthermore, previous studies on the improvement of the therapeutic efficacy of EGFR/HER2-targeted therapies through ANO1 inhibition suggest that the compound according to the present invention may enhance the responsiveness of brain tumor cells to EGFR/HER-targeted therapy and suppress the development of resistance.

In particular, the compound according to the present invention simultaneously inhibits the expression of EGFR together with ANO1 in brain tumor cells, and thus may exhibit a stronger anticancer effect through concurrent inhibition of ANO1 and EGFR. The association between ANO1 and EGFR is well established, as upregulation of ANO1 is related to the signaling pathway of EGFR and has been reported to have a significant effect on the remodeling of the phosphorylated proteome following epidermal growth factor (EGF) stimulation. In addition, in cancer cells, ANO1 may form a functional complex with EGFR to cooperatively regulate cell proliferation. Therefore, when EGFR and ANO1 are simultaneously inhibited by using the compound according to the present invention, the growth of brain tumors may be more effectively suppressed.

Meanwhile, ANO1 is known to be overexpressed in cancer cells and to contribute to cancer cell migration and tumor metastasis, and signaling through EGFR is also known to create a tumor microenvironment that promotes tumor metastasis. Accordingly, the compound according to the present invention may effectively inhibit the metastasis of brain tumors through simultaneous inhibition of ANO1 and EGFR. The term "metastasis" as used herein refers to a condition in which a malignant tumor has spread from the organ of origin to another distant tissue. Therefore, the composition according to the present invention may inhibit the metastasis of brain tumors and thereby prevent and treat the systemic spread of brain tumors.

The compound according to the present invention or pharmaceutically acceptable salts thereof may be used for the prevention or treatment of various cancers.

In the present invention, the term "tumor" is used synonymously with "cancer" and refers to a condition typically characterized by uncontrolled cell growth, migration, and proliferation. Preferably, the cancer according to the present invention is a brain tumor. The cancer according to the present invention includes both primary and recurrent cancers and encompasses both benign and malignant tumors. In the present invention, the brain tumor refers to any tumor occurring within the cranium, and is not limited to a specific type, but includes all tumors arising in the brain and surrounding structures thereof. In the present invention, the brain tumor includes primary brain tumors, recurrent brain tumors, and metastatic brain tumors. In addition, the brain tumor includes astrocytoma, malignant astrocytoma, glioblastoma, meningioma, pituitary adenoma, schwannoma, acoustic schwannoma, and craniopharyngioma, and is not limited to any particular type.

In particular, the cancer according to the present invention may be a brain tumor expected to be improved or treated by simultaneously inhibiting ANO1 and EGFR. Preferably, the cancer according to the present invention may be a brain tumor associated with one or more mutations selected from the group consisting of EGFR (epidermal growth factor receptor) and ANO1 (Anoctamin 1). That is, the cancer according to the present invention may be a brain tumor having a mutation in EGFR and/or ANO1. The mutation of EGFR and/or ANO1 includes amplification of the EGFR and/or ANO1 gene, protein overexpression, protein hyperactivation, and continuous activation of the protein. That is, the cancer according to the present invention may be a brain tumor in which the expression or activity of EGFR and/or ANO1 is higher than that of normal cells. Alternatively, the cancer according to the present invention may be a brain tumor in which the activation state of EGFR and/or ANO1 is sustained. Since the compound according to the present invention is capable of simultaneously inhibiting the expression and activity of ANO1 and EGFR, it may exhibit particularly excellent anticancer effects against brain tumors involving mutations in EGFR and/or ANO1.

Alternatively, the cancer according to the present invention may be a brain tumor associated with an EGFRvIII (epidermal growth factor receptor variant III) mutation. The EGFRvIII mutation is a mutant form in which amino acids 6 to 273 (or exons 2 to 7) of EGFR are deleted, and is characterized in that it remains in an activated state without binding of a ligand (EGF). EGFRvIII is specifically expressed only in cancer cells and is found in approximately 30% of glioblastomas.

The compound according to the present invention may exhibit one or more effects selected from the group consisting of the following:
(a) inhibiting proliferation or growth of brain tumor cells;
(b) inhibiting migration of brain tumor cells;
(c) inhibiting metastasis of brain tumors;
(d) suppressing resistance of brain tumors to anticancer agents; and
(e) enhancing responsiveness of brain tumor cells to anticancer agents.

The inventors confirmed through specific examples that brain tumor cells treated with the compound according to the present invention exhibited inhibited proliferation, migration, and metastasis (invasiveness), and the anticancer effect of the compound was found to be stronger than that of the conventional ANO1 inhibitor (CaCCinh-A01).

Furthermore, since the compound according to the present invention can simultaneously inhibit ANO1 and EGFR (that is, dual inhibition), it may exhibit a superior anticancer effect compared to single inhibitors of ANO1 or EGFR, and in particular, by inhibiting ANO1, it may suppress the development of resistance of cancer cells to anticancer agents and enhance the anticancer efficacy of the anticancer agents. For example, the inventors confirmed through experiments that the ANO1 inhibitory effect of the compound according to the present invention was superior to that of the conventional ANO1 inhibitor CaCCinh-A01, and that its anticancer effect was superior to those of the ANO1 inhibitor CaCCinh-A01, the EGFR inhibitor Osimertinib, and the combination of Osimertinib and CaCCinh-A01.

In one embodiment of the present invention, the anticancer agent described in (d) and (e) may be a targeted anticancer agent for tyrosine kinase, but is not limited thereto. As used herein, the term "targeted anticancer agent" refers to an agent that exerts an anticancer effect by targeting proteins or genes specifically altered in cancer cells or cancer tissues and interfering with molecular activities involved in the growth and development of cancer. Preferably, the tyrosine kinase may be one or more selected from the group consisting of EGFR (epidermal growth factor receptor), ALK (anaplastic lymphoma kinase), ROS1 (ROS Proto-Oncogene 1), BRAF (B-Raf Proto-Oncogene), HER2 (human epidermal growth factor receptor 2), RET (Ret Proto-Oncogene), NTRK1 (Neurotrophic Receptor Tyrosine Kinase 1), MET (Mesenchymal-Epithelial Transition factor), and NRG1 (Neuregulin 1). More preferably, the tyrosine kinase may be EGFR. The EGFR includes normal EGFR and mutant EGFR (for example, EGFRvIII).

The enhancement of cancer cell responsiveness to anticancer agents as described in (e) means that the inhibition of cancer cell growth and migration by the anticancer agent is further enhanced.

In the present invention, the term "enhancing anticancer efficacy" refers to all effects that can ultimately enhance the function of an anticancer agent, and includes not only enhancing the anticancer effects of the anticancer agent such as inhibition of tumor growth, inhibition of tumor metastasis, and inhibition of tumor recurrence, but also enhancing the anticancer effect as a result of suppressing resistance or tolerance of cancer cells to the anticancer agent.

Accordingly, the compound according to the present invention may be used as a compound for combination therapy with a known anticancer agent (preferably, a targeted anticancer agent for tyrosine kinase) for the purpose of enhancing the anticancer efficacy of a brain tumor anticancer agent.

For example, the present invention provides a pharmaceutical composition for preventing or treating cancer (preferably a brain tumor), comprising (i) one or more compounds selected from the group consisting of the compounds represented by Chemical Formulae 1 to 6 or pharmaceutically acceptable salts thereof; and (ii) a targeted anticancer agent for EGFR as active ingredients.

In addition, the present invention provides a pharmaceutical composition for enhancing the anticancer efficacy of an anticancer agent (preferably, a brain tumor anticancer agent), comprising, as an active ingredient, one or more compounds selected from the group consisting of the compounds represented by Chemical Formulae 1 to 6 or pharmaceutically acceptable salts thereof.

The composition according to the present invention may be in the form of a mixture in which the compound or pharmaceutically acceptable salts thereof and the targeted anticancer agent for EGFR are mixed, and may be a formulation for simultaneous administration of the compound or pharmaceutically acceptable salts thereof and the targeted anticancer agent for EGFR.

In addition, the composition according to the present invention may be in a form in which the compound or pharmaceutically acceptable salts thereof and the targeted anticancer agent for EGFR are each formulated and administered simultaneously, separately, or sequentially. In this case, the composition may be a pharmaceutical composition for combination therapy for simultaneous or sequential administration, comprising a first pharmaceutical composition containing a pharmaceutically effective amount of the compound or a salt thereof as an active ingredient, and a second pharmaceutical composition containing a pharmaceutically effective amount of the targeted anticancer agent for EGFR as an active ingredient. In this case, in sequential administration, the order of administration is not limited, and the administration regimen may be appropriately adjusted according to the condition of the patient or the like.

That is, when the pharmaceutical composition is a pharmaceutical composition for combination therapy for sequential administration, the composition may be such that the compound or a salt thereof ("first component") is administered first, followed by administration of the targeted anticancer agent for EGFR ("second component"), and the reverse order is also possible.

In addition, when the compound according to the present invention is administered in combination with an anticancer agent, the compound may be administered simultaneously, separately, or sequentially with the anticancer agent, and even in the case of sequential administration, the order of administration is not limited, and the administration regimen may be appropriately adjusted depending on the type of cancer, type of anticancer agent, or the condition of the patient.

The content of the compound in the composition of the present invention may be appropriately adjusted depending on the symptoms of the disease, the degree of progression of the symptoms, and the condition of the patient, and for example, may be 0.0001 to 99.9% by weight, or 0.001 to 50% by weight, based on the total weight of the composition, but is not limited thereto. The content ratio is based on the dry weight obtained after removal of the solvent.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the age, sex, and body weight of the patient, and generally, 0.001 to 150 mg, preferably 0.01 to 100 mg, per kg of body weight may be administered daily, every other day, or divided into 1 to 3 doses per day. However, since the dosage may be increased or decreased depending on the route of administration, severity of the disease, sex, body weight, and age, the above dosage in no way limits the scope of the present invention.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

In addition, the present invention provides a kit for preventing or treating cancer, comprising the pharmaceutical composition according to the present invention.

The kit according to the present invention may further comprise, in addition to the compound and the targeted anticancer agent, other components, compositions, solutions, or devices commonly required for the prevention or treatment of cancer without limitation, and may particularly include an instruction manual indicating proper use and storage of the compound according to the present invention.

The terms and words used in the present specification and claims shall not be construed as being limited to their ordinary or dictionary meanings, but shall be interpreted in accordance with the meanings and concepts consistent with the technical spirit of the present invention, based on the principle that the inventor can properly define the concepts of the terms in order to best describe his own invention.

### [Mode for Invention]

Hereinafter, preferred examples are presented to help understand the present invention. However, the following examples are provided only to help understand the present invention more easily, and the contents of the present invention are not limited by the following examples.

### [EXAMPLES]

### Example A: Novel compounds AON-MG23-01 and AON-MG23-02

### 1. Preparation of compounds according to the present invention

### 1-1. Preparation of N-(2-((5-chloro-2-((2-methoxy-4-(4-(4-methylpiperazin-1-yl)piperidin-1-yl)phenyl)amino)pyrimidin-4-yl)amino)phenyl)-N-cyclopropylmethanesulfonamide (AON-MG23-01)

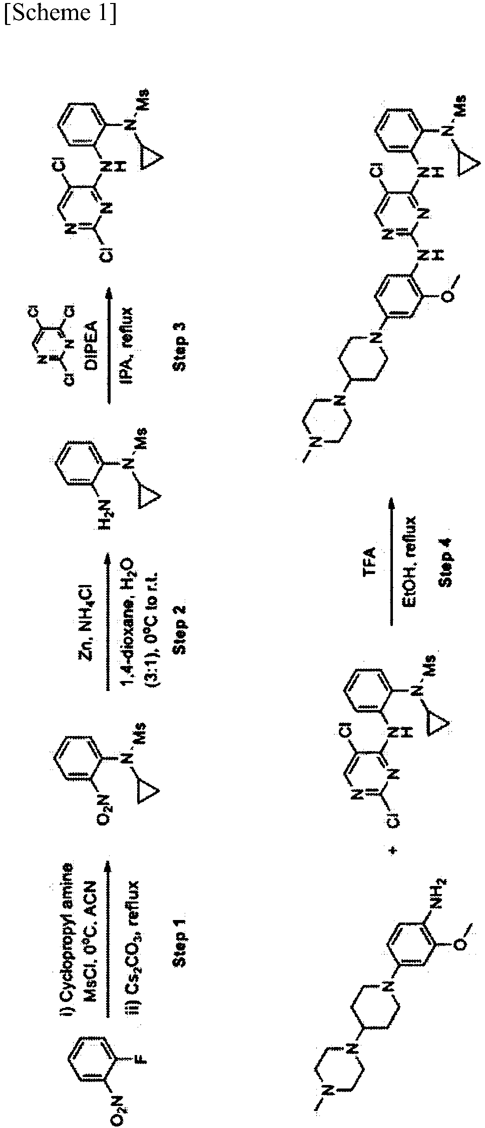

An AON-MG23-01 compound was prepared through Scheme 1 above. The specific preparation method is as follows:

### <Step 1> Synthesis of N-cyclopropyl-N-(2-nitrophenyl)methanesulfonamide

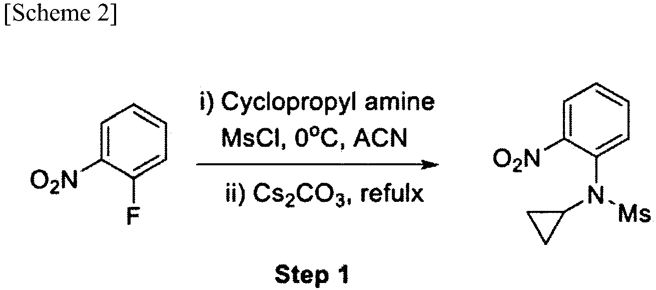

1-Fluoro-2-nitrobenzene (1.00 eq) and cyclopropyl amine (1.00 eq) were dissolved in acetonitrile (ACN), and methanesulfonyl chloride (MsCl, 1.0 eq) was slowly added at 0°C, and then stirred at the same temperature for an hour. Thereafter, cesium carbonate (Cs₂CO₃, 5.00 eq) was added at the same temperature, refluxed, and stirred overnight. After the reaction was completed, the temperature was lowered to room temperature, and the resulting mixture was extracted using ethyl acetate and water. The organic layer was stirred after adding anhydrous sodium sulfate, and filtered using a filter, and the filtrate was then concentrated under reduced pressure. The concentrated residue was purified by column chromatography (hexane:ethyl acetate = 3:1) to synthesize N-cyclopropyl-N-(2-nitrophenyl)methanesulfonamide as a light yellow solid in a yield of 36%.

### <Step 2> Synthesis of N-(2-aminophenyl)-N-cyclopropylmethanesulfonamide

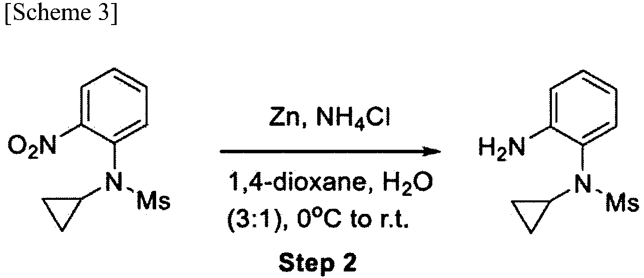

The N-cyclopropyl-N-(2-nitrophenyl)methanesulfonamide (1.00 eq) synthesized in Step 1 was added to 1,4-dioxane and water (3:1), and stirred, and the reactor was then cooled to 0°C, and zinc (Zn, 10.0 eq) and ammonium chloride (NH₄Cl, 10.0 eq) were added thereto. Thereafter, the resulting mixture was stirred for 4 hours while gradually increasing the temperature. After the reaction was completed, the mixture was filtered through Celite, and the filtrate was extracted with ethyl acetate and water. The organic layer was stirred after adding anhydrous sodium sulfate, and filtered using a filter, and the filtrate was then concentrated under reduced pressure. The concentrated residue was used in Step 3 without any additional purification.

### <Step 3> Synthesis of N-cyclopropyl-N-(2-((2,5-dichloropyrimidine-4-yl)amino)phenyl)methanesulfonamide

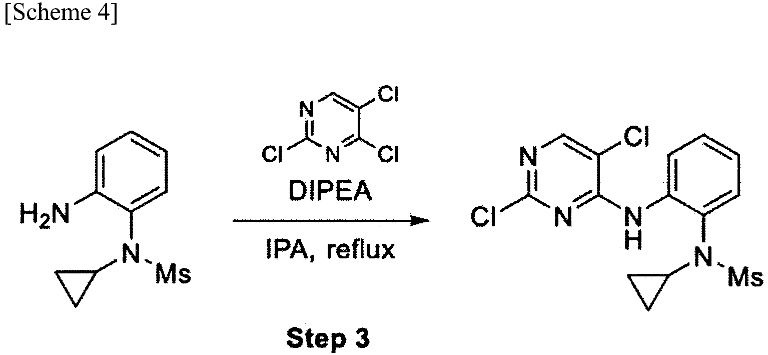

The N-(2-aminophenyl)-N-cyclopropylmethanesulfonamide (1.00 eq) synthesized in Step 2 was added to isopropyl alcohol (IPA), and 2,4,5-trichloropyrimidine (1.1 eq) and N,N-diisopropylethylamine (DIPEA, 2.5 eq) were added at room temperature. Thereafter, the resulting mixture was refluxed and stirred overnight. After the reaction was completed, the mixture was evaporated under reduced pressure, and extracted with water and dichloromethane. The organic layer was washed with 2 N hydrochloric acid, and then stirred after adding anhydrous sodium sulfate. Thereafter, the resulting mixture was filtered using a filter, and the filtrate was concentrated under reduced pressure. The concentrated residue was purified by column chromatography (hexane:ethyl acetate = 3:1) to synthesize N-cyclopropyl-N-(2-((2,5-dichloropyrimidin-4-yl)amino)phenyl)methanesulfonamide as a light yellow solid in a yield of 58.9%.

### <Step 4> Synthesis of N-(2-((5-chloro-2-((2-methoxy-4-(4-(4-methylpiperazin-1-yl)piperidine-1-yl)phenyl)amino)pyrimidine-4-yl)amino)phenyl)-N-cyclopropylmethanesulfonamide

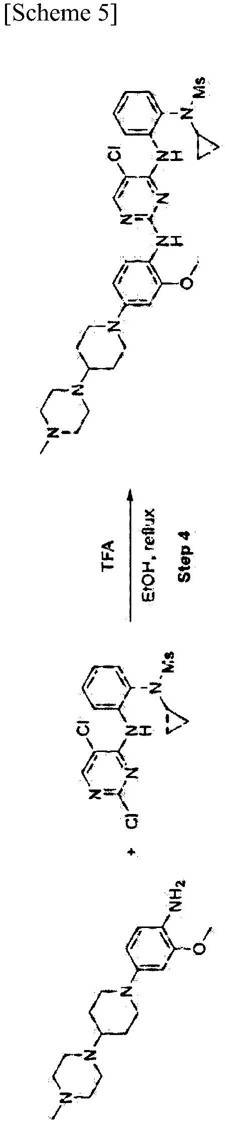

The N-cyclopropyl-N-(2-((2,5-dichloropyrimidin-4-yl)amino)phenyl)methanesulfonamide (1.00 eq) synthesized in Step 3 was added to ethanol (EtOH), and 2-methoxy-4-(4-(4-methylpiperazin-1-yl)piperidin-1-yl)aniline (1.00 eq) and trifluoroacetic acid (TFA, 1.95 eq) were added at room temperature. Thereafter, the resulting mixture was refluxed and stirred overnight. After the reaction was completed, the mixture was neutralized with a 1 N sodium hydroxide solution, and extracted with water and ethyl acetate. The organic layer was stirred after adding anhydrous sodium sulfate, and filtered using a filter, and the filtrate was then concentrated under reduced pressure. The concentrated residue was purified by column chromatography (hexane:ethyl acetate = 3:1) to synthesize N-(2-((5-chloro-2-((2-methoxy-4-(4-(4-methylpiperazin-1-yl)piperidin-1-yl)phenyl)amino)pyrimidin-4-yl)amino)phenyl)-N-cyclopropylmethanesulfonamide (AON-MG23-01) as a yellow solid in a yield of 14%.

H NMR (500 MHz, DMSO-d6) δ 8.33 (s, 1H), 8.19 (s, 1H), 8.09 (s, 1H), 7.99 (s, 1H), 7.62 (dd, J = 7.9, 1.6 Hz, 1H), 7.35 (d, J = 8.7 Hz, 1H), 7.23 (t, J = 7.8 Hz, 1H), 7.15 (td, J = 7.6, 1.5 Hz, 1H), 6.62 (d, J = 2.5 Hz, 1H), 6.47 (dd, J = 8.7, 2.5 Hz, 1H), 3.75 - 3.71 (m, 5H), 3.25 - 3.22 (m, 4H), 2.67 (td, J = 12.2, 2.4 Hz, 2H), 2.55 - 2.44 (m, 4H), 2.38 - 2.27 (m, 4H), 2.15 (s, 3H), 1.85 (d, J = 11.5 Hz, 2H), 1.52 (qd, J = 12.1, 3.9 Hz, 2H), 1.01 - 0.93 (m, 2H), 0.55 - 0.49 (m, 1H), 0.17 - 0.12 (m, 1H). HRMS: 640.2708, cal: 640.2711

### 1-2. Preparation of N-(2-((5-chloro-2-((4-(4-(dimethylamino)piperidin-1-yl)-2-methoxyphenyl)amino)pyrimidin-4-yl)amino)phenyl)-N-cyclopropylmethanesulfonamide (AON-MG23-02)

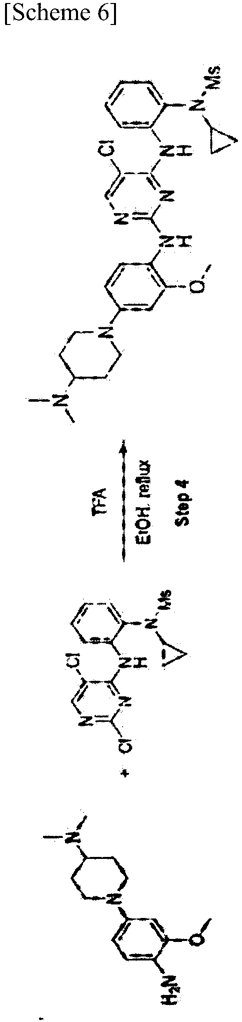

Steps 1, 2, and 3 were performed in the same manner as for the AON-MG23-01 compound, and synthesis in Step 4 was performed using 1-(4-amino-3-methoxyphenyl)-N,N-dimethylpiperidine-4-amine instead of 2-methoxy-4-(4-(4-methylpiperazin-1-yl)piperidine-1-yl)aniline.

Specifically, the N-cyclopropyl-N-(2-((2,5-dichloropyrimidin-4-yl)amino)phenyl)methanesulfonamide (1.00 eq) synthesized in Step 3 was added to ethanol (EtOH), and 1-(4-amino-3-methoxyphenyl)-N,N-dimethylpiperidin-4-amine (1.00 eq) and trifluoroacetic acid (TFA, 1.95 eq) were added at room temperature. Thereafter, the resulting mixture was refluxed and stirred overnight. After the reaction was completed, the mixture was neutralized with a 1 N sodium hydroxide solution, and extracted with water and ethyl acetate. The organic layer was stirred after adding anhydrous sodium sulfate, and filtered using a filter, and the filtrate was then concentrated under reduced pressure. The concentrated residue was purified by column chromatography (hexane:ethyl acetate = 3:1) to synthesize N-(2-((5-chloro-2-((4-(4-(dimethylamino)piperidin-1-yl)-2-methoxyphenyl)amino)pyrimidin-4-yl)amino)phenyl)-N-cyclopropylmethanesulfonamide (AON-MG23-02) as a yellow solid in a yield of 25%.

¹H NMR (500 MHz, DMSO-d6) δ 8.33 (s, 1H), 8.19 (s, 1H), 8.09 (s, 1H), 7.99 (s, 1H), 7.62 (dd, J = 7.8, 1.6 Hz, 1H), 7.35 (d, J = 8.7 Hz, 1H), 7.22 (d, J = 8.1 Hz, 1H), 7.15 (td, J = 7.6, 1.6 Hz, 1H), 6.63 (d, J = 2.5 Hz, 1H), 6.48 (dd, J = 8.7, 2.5 Hz, 1H), 3.75 (s, 3H), 3.72 (d, J = 12.2 Hz, 2H), 3.23 (m, 4H), 2.68 (td, J = 12.1, 2.5 Hz, 2H), 2.22 (s, 6H), 1.86 (d, J = 12.4 Hz, 2H), 1.51 (qd, J = 12.0, 3.9 Hz, 2H), 1.04 - 0.92 (m, 2H), 0.55 - 0.49 (m, 1H), 0.17 - 0.14 (m, 1H). HRMS: 585.2294, cal: 585.2289.

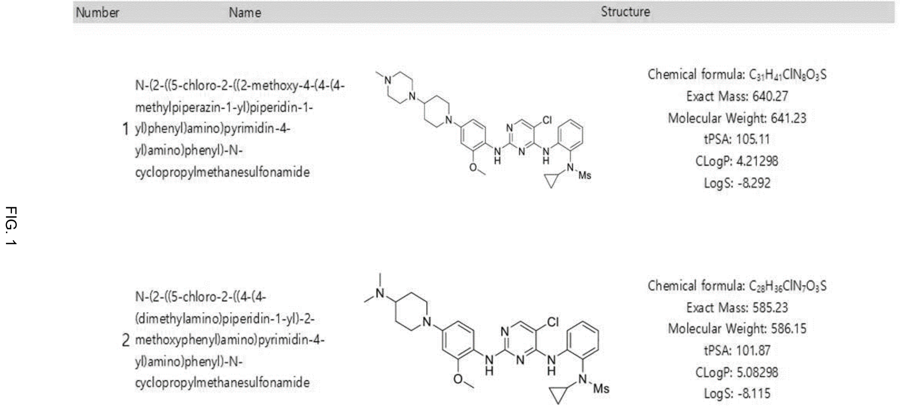

The compounds according to the present invention were prepared through the above steps, and their structural formulas are shown in Table 1 and FIG. 1 below.

**[Table 1]**

| Classification | Name | Structure |
|---|---|---|
| AON-MG23-01 | N-(2-((5-chloro-2-((2-methoxy-4-(4-(4-m ethylpiperazin-1-yl)piperidin-1-yl)phenyl) amino)pyrimidin-4-yl)amino)phenyl)-N-c yclopropylmethanesulfonamide | 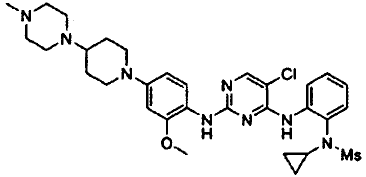 |
| AON-MG23-02 | N-(2-((5-chloro-2-((4-(4-(dimethylamino) piperidin-1-yl)-2-methoxyphenyl)amino)p yrimidin-4-yl)amino)phenyl)-N-cycloprop ylmethanesulfonamide | 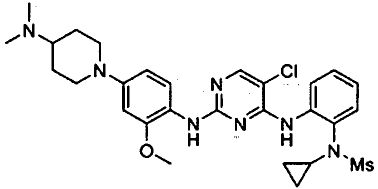 |

### 2. Confirmation of cell proliferation inhibitory effect of compound of the present invention on human glioblastoma cells

Human glioblastoma cell line U87-MG cells were seeded at a density of 1 X 10⁴ cells/well in a 96-well plate and cultured for 24 hours. Thereafter, the cells were treated with DMSO as a control and the compound AON-MG23-01 or AON-MG23-02 of the present invention, and then cultured for 48 hours. The compound of the present invention was treated at various concentrations of 0, 1, 2, and 4 µM. 10 µL of a CCK reagent was added and reacted for an hour, and the absorbance was then measured at 450 nm.

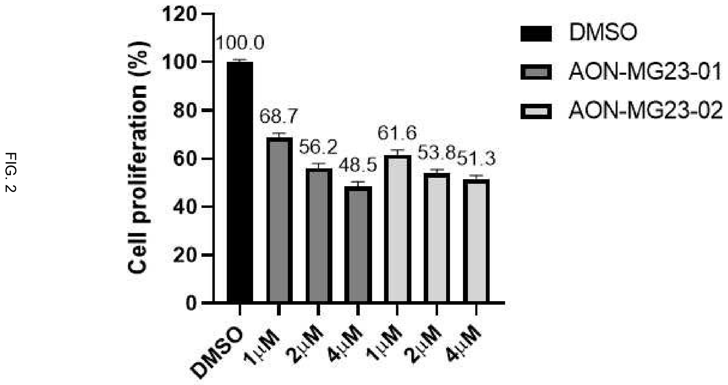

As a result, as shown in FIG. 2, it can be seen that the compound AON-MG23-01 or AON-MG23-02 of the present invention inhibits the proliferation of U87-MG cells.

### 3. Confirmation of cell migration inhibitory effect of compound of the present invention on human glioblastoma cells

Human glioblastoma cell line U87-MG cells were seeded at a density of 8 X 10⁴ cells/well on a SPLScar Block (SPL) plate and cultured for 24 hours. After 24 hours, the block was removed, and the cells were treated with the compound AON-MG23-01 or AON-MG23-02 of the present invention, and cultured at 37°C in a CO₂ incubator for 24 hours. The compound of the present invention was treated at concentrations of 0, 1, 2, and 4 µM. Images were taken before and after culture, and the distance of cell migration was measured using Image J software. Based on the results, whether the cell migration was inhibited was analyzed.

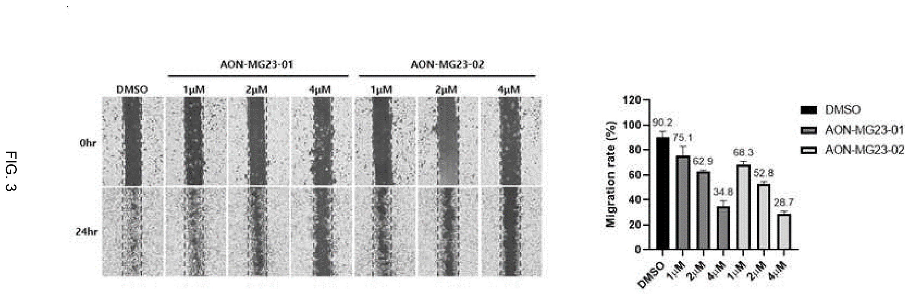

As a result, as shown in FIG. 3, it was confirmed that when the compound AON-MG23-01 or AON-MG23-02 of the present invention was treated at a concentration of 4 µM, the cell migration was inhibited by more than 65% compared to the untreated control.

### 4. Confirmation of metastasis inhibitory effect of compound of the present invention on human glioblastoma

A human glioblastoma cell line U87-MG was seeded at a density of 8 X 10⁴ cells/well on Matrigel-coated filters (Transwell invasion chambers, Corning), and the cells were then treated with the compound (AON-MG23-01 or AON-MG23-02; each at 0, 1, 2, and 4 µM) of the present invention, and cultured at 37°C in a CO₂ incubator for 24 hours. After the culturing was completed, the cells were fixed and stained using the Diff-Quick Stain kit (Sysmex, Kobe, Japan). Images of the stained cells were taken under a microscope, the cells were counted using Image J software, and statistical analysis was then performed.

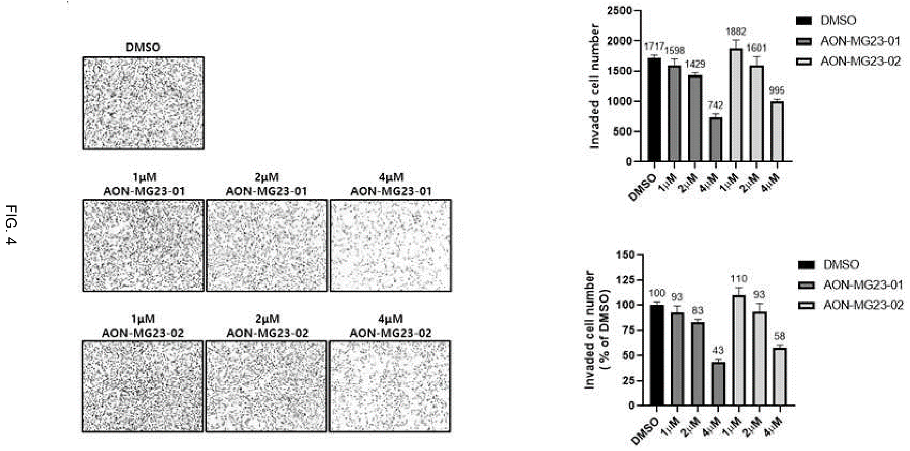

As a result, as shown in FIG. 4, it can be seen that the group treated with the compound AON-MG23-01 or AON-MG23-02 of the present invention showed a reduced degree of penetration into Matrigel compared to the untreated control, indicating that the metastatic ability of cancer cells was suppressed.

### 5. Confirmation of effect of compound of the present invention on ANO1 and EGFR protein expression in glioblastoma cell line

It was determined whether the compound of the present invention affects the expression of ANO1 and EGFR proteins in a glioblastoma cell line. The human glioblastoma cell line U87-MG was seeded at a density of 1 X 10⁶ cells in a 60 mm dish, and cultured for 24 hours, and the cells were then treated with the compound (AON-MG23-01 or AON-MG23-02; each at 0, 1, 2, and 4 µM) of the present invention, and cultured for 48 hours. The cultured cells were harvested and the harvested cells were lysed for Western blotting. An anti-ANO1 antibody (Abcam, ab53212), an anti-pEGFR antibody (Cell Signaling, #4407S), an anti-EGFR antibody (Cell Signaling, #4267S), and an anti-α-tubulin antibody (Santacruz, #SC-5286) were used as the antibodies for Western blotting. Protein concentration was quantified using the BSA method (a protein quantitation assay, a bovine serum albumin assay) (Pierce, Cat. 23225). After the cells were lysed, an equal amount (10 µg) of the proteins obtained from the lysed cells was subjected to 10% SDS-PAGE to separate the proteins depending on their molecular weights, transferred to a PVDF membrane (Bio-Rad), and then treated with a blocking buffer (5% skim milk in a Tris-buffered saline (TBS) buffer containing 0.1% Tween 20; TBS-T) at room temperature for an hour. Thereafter, the PVDF membrane was treated with the primary antibody, and reacted at 4°C for 16 hours. The reacted PVDF membrane was washed three times with TBS-T. The PVDF membrane was then reacted with a horseradish peroxidase-labeled secondary antibody at room temperature for an hour, and then visualized using an ECL kit (Bio-rad). The amount of each protein was quantified and analyzed using Image J software.

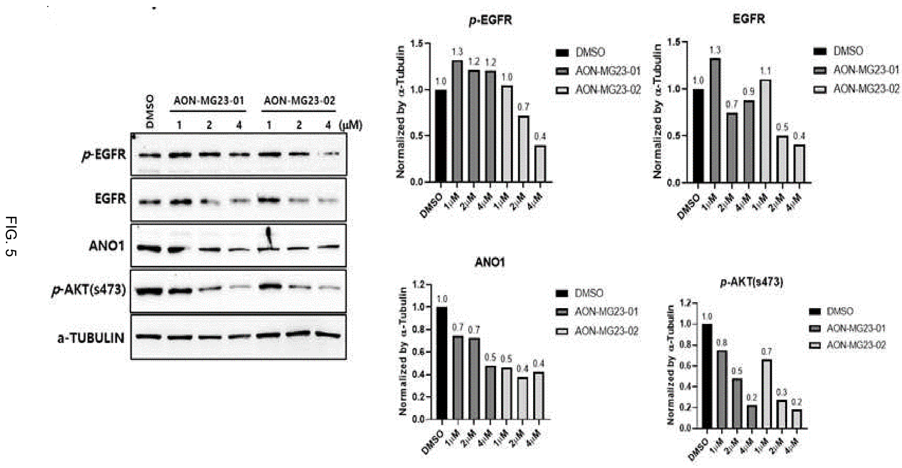

As a result, as shown in FIG. 5, the protein levels of ANO1 and EGFR were significantly inhibited in the cells treated with the compound of the present invention compared to the untreated control, and this protein expression inhibitory effect was dependent on the treatment concentration of the compound.

### 6. Confirmation of inhibitory effect of compound of the present invention on ANO1 activity in glioblastoma cell line

The inhibitory effect on ANO1 ion channel activity was confirmed by measuring calcium-dependent chloride ion channel activity by the compound of the present invention using a patch clamp. For this purpose, patch clamping was performed on U87-MG cells expressing ANO1. A pipette solution was composed of: 146 CsCl, 5 Ca-EGTA-NMDG, 8 HEPES, 2 MgCl₂, and 10 sucrose (pH 7.3), and a bath solution was composed of: 50 NaCl, 10 HEPES, 3 KCl, 2 CaCl₂, 2 MgCl₂, and 5.5 glucose (pH 7.3). U87-MG cells were cultured on a coverslip for 4 hours, and then treated with AON-MG23-01 or AON-MG23-02 for an hour (at concentrations of 0, 4, and 8 µM). At this time, the chloride ion channels were activated using 400 mM ATP and high-concentration calcium, and calcium-dependent chloride ion channel currents in the range of -100 mV to +100 mV were measured.

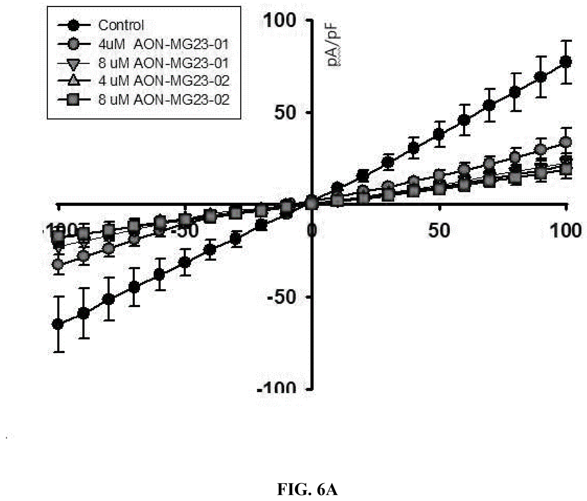

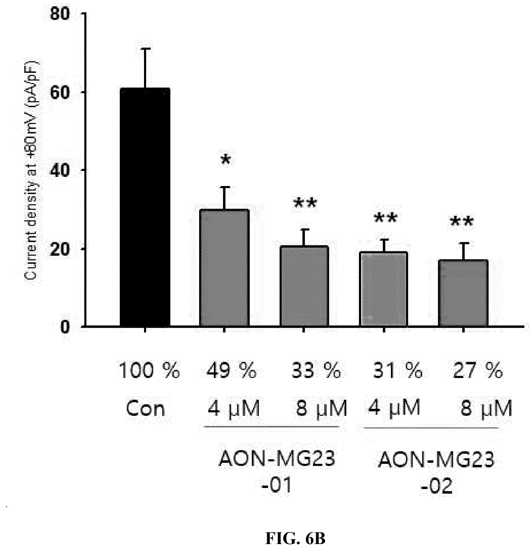

As a result, as shown in FIG. 6A, it was confirmed that the group treated with the compound of the present invention showed a significant decrease in ANO1 activity compared to the untreated control. In particular, as shown in FIG. 6B, it was confirmed that the group treated with the compound of the present invention showed a decrease in current density of more than 50% at a membrane potential of +80 mV compared to the untreated control. The results demonstrate that the compound of the present invention can inhibit the activity of ANO1, which is a calcium-dependent chloride ion channel, in glioblastoma cell line U87-MG cells.

### 7. Comparison of metastasis inhibitory effects of compound of the present invention and EGFR inhibitor and ANO1 inhibitor in human glioblastoma

A human glioblastoma cell line U87-MG was seeded at a density of 6 X 10⁴ cells/well on the top of a Matrigel-coated filter (Transwell invasion chambers, Corning), treated with 4 µM of the compound AON-MG23-02 of the present invention, 4 µM of the EGFR inhibitor Osimertinib, 100 µM of the ANO1 inhibitor CaCCinh-A01, or a combination of 4 µM of Osimertinib and 100 µM of CaCCinh-A01, and then cultured at 37°C in a CO₂ incubator for 16 hours. After the culturing was completed, the cells were fixed and stained using a Diff-Quick stain kit (Sysmex, Kobe, Japan). Images of the stained cells were taken under a microscope, the cells were counted using Image J software, and statistical analysis was then performed.

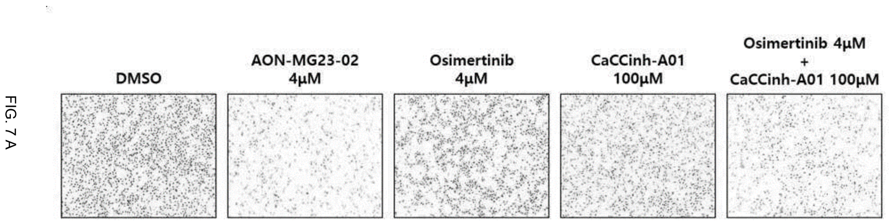

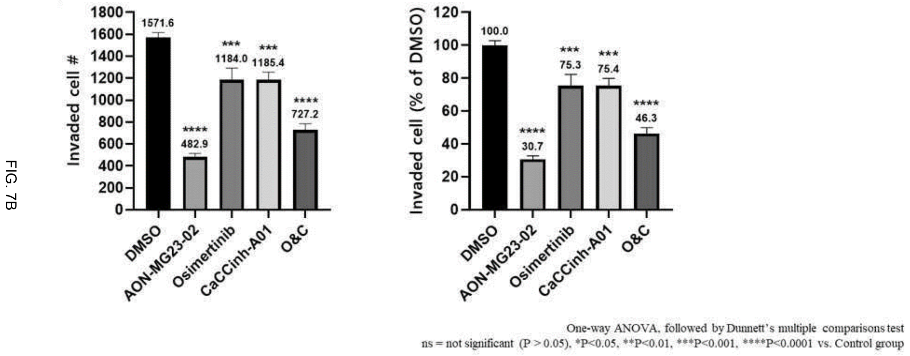

As a result, as shown in FIGS. 7A and 7B, the group treated with the compound AON-MG23-02 of the present invention showed a reduced degree of penetration into Matrigel compared to the untreated control, the EGFR inhibitor-treated group, and the ANO1 inhibitor-treated group, and has a metastasis inhibitory effect similar to that of the EGFR inhibitor and ANO1 inhibitor combination-treated group.

### 8. Comparison of inhibitory effects of compound of the present invention and EGFR inhibitor and ANO1 inhibitor on expression of ANO1, EGFR, and pEGFR proteins in glioblastoma cell line

The inhibitory effects of the compound AON-MG23-02 of the present invention, the EGFR inhibitor Osimertinib, and the ANO1 inhibitor CaCCinh-A01 on the expression of ANO1, EGFR, and pEGFR proteins in a glioblastoma cell line were compared. A human glioblastoma cell line U87-MG was seeded at a density of 100 X 10⁴ cells/dish in a 60 mm dish, and cultured for 24 hours. Thereafter, the cells were treated with 4 µM of AON-MG23-02, 2 µM of the EGFR inhibitor Osimertinib, 100 µM of the ANO1 inhibitor CaCCinh-A01, or a combination of 2 µM of Osimertinib and 100 µM of CaCCinh-A01, and cultured for 48 hours. The cultured cells were harvested and the harvested cells were lysed for Western blotting. An anti-ANO1 antibody (Abcam, ab53212), an anti-pEGFR antibody (Cell Signaling, #4407S), an anti-EGFR antibody (Cell Signaling, #4267S), and an anti-ACTIN antibody (sigma, #A2066) were used as antibodies for Western blotting. Protein concentration was quantified using the BSA method (a protein quantitation assay, a bovine serum albumin assay) (Pierce, Cat. 23225). After the cells were lysed, an equal amount (10 µg) of the proteins obtained from the lysed cells was subjected to 10% SDS-PAGE to separate the proteins depending on their molecular weights, transferred to a PVDF membrane (Bio-rad), and then treated with a blocking buffer (5% skim milk in a Tris-buffered saline (TBS) buffer containing 0.1% Tween 20; TBS-T) at room temperature for an hour. Next, the PVDF membrane was treated with the primary antibody and reacted at 4°C for 16 hours. The reacted PVDF membrane was washed three times with TBS-T for 10 minutes. The PVDF membrane was then reacted with the horseradish peroxidase-labeled secondary antibody at room temperature for an hour, and then washed three times with TBS-T for 10 minutes. The washed PVDF membrane was treated with the ECL kit (Thermo, West Pico Plus), reacted, and visualized using the da Vinci-Q (Youngin Lab Plus) device. The amount of each protein was quantified and analyzed using Image J software.

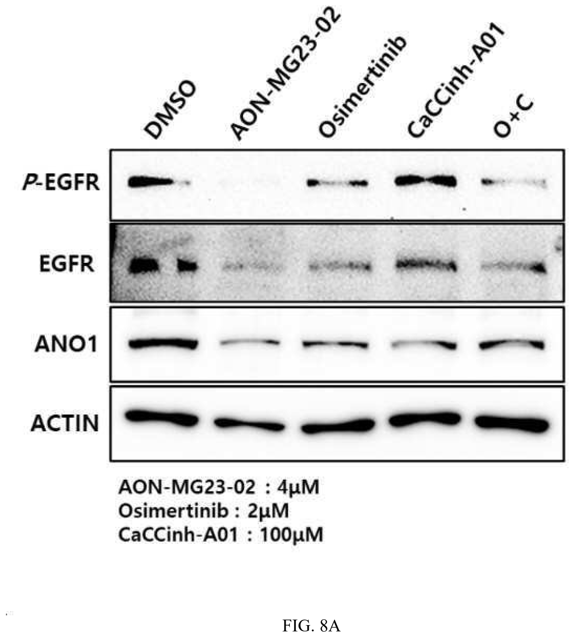

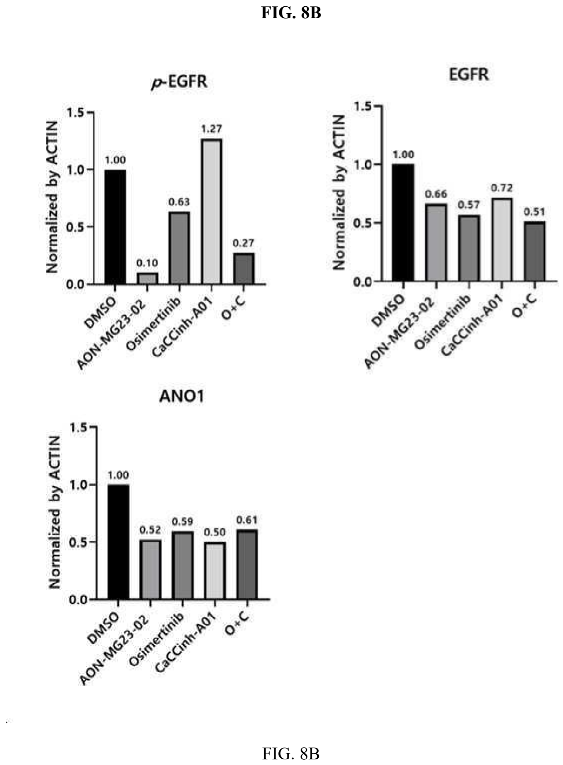

As a result, as shown in FIGS. 8A and 8B, it was confirmed that the compound AON-MG23-02 of the present invention reduces the expression of ANO1, EGFR, and pEGFR proteins compared to the untreated control, the EGFR inhibitor-treated group, the ANO1 inhibitor-treated group, and the EGFR inhibitor and ANO1 inhibitor combination-treated group.

### Example B. Derivatives of novel compound AON-MG23-02

### 1. Preparation of derivatives of AON-MG23-02

### 1-1. AON-MG23-05

### 1-1-A. Preparation of Compound 2

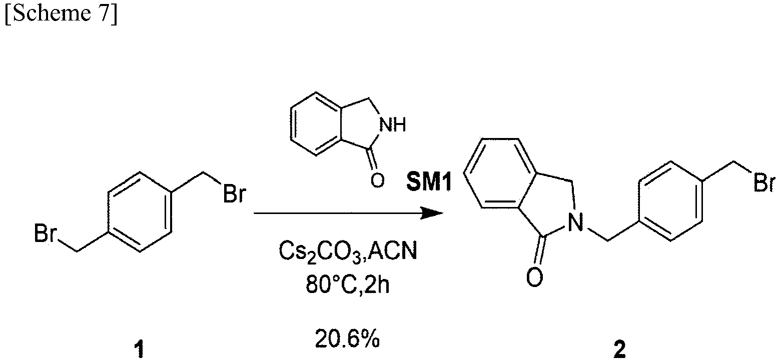

Compound 1 (3.00 g, 11.3 mmol, 1.00 eq), the compound SM1 (756 mg, 5.68 mmol, 0.500 eq), and Cs₂CO₃ (2.96 g, 9.09 mmol, 0.800 eq) were mixed in acetonitrile (ACN) (10.0 mL). The resulting mixture was degassed, purged three times with N₂, and then stirred at 80°C in nitrogen gas (N₂) for 2 hours. TLC (petroleum ether:ethyl acetate = 1:1, R_{f} = 0.49) analysis results showed that the compound SM1 was completely consumed and a large amount of new spots were generated. The reaction mixture was concentrated under reduced pressure. The residue was diluted with 6.00 mL of water, and then extracted with 6.00 mL of EtOAc (6.00 mL * 3). The mixed organic supernatant was washed with 9.00 mL of brine (9.00 mL * 1), dried over Na₂SO₄, filtered, and then concentrated under reduced pressure to obtain a residue. The residue was purified by column chromatography (SiO₂, petroleum ether:ethyl acetate = 20:1 to 0:1). Compound 2 (900 mg, 2.35 mmol, yield: 20.6%, and purity: 82.4%) was obtained as a yellow solid.

### 1-1-B. Preparation of Compound 3

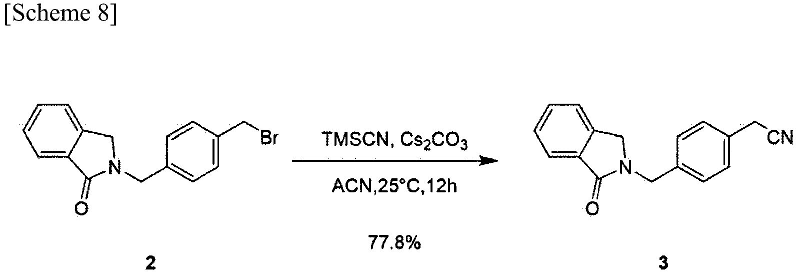

Trimethylsilyl cyanide (TMSCN, 338 mg, 3.42 mmol, 427 µL, 1.20 eq) and Cs₂CO₃ (1.11 g, 3.42 mmol, 1.20 eq) were added to a solution of Compound 2 (900 mg, 2.85 mmol, 1.00 eq) dissolved in ACN (10.0 mL). The mixture was stirred at 25°C for 12 hours. LC-MS analysis results showed that Compound 2 was completely consumed, and 42.0% of the desired mass was detected. The reaction mixture was concentrated under reduced pressure, and the residue was diluted with 2.00 mL of water, and then extracted with 3.00 mL of EtOAc (3.00 mL * 3). The combined organic supernatant was washed with 4.00 mL of brine (4.00 mL * 1), dried over Na₂SO₄, filtered, and then concentrated under reduced pressure to obtain a residue. The residue was purified by column chromatography (SiO₂, petroleum ether:ethyl acetate = 10:1 to 0:1). Compound 3 (600 mg, 2.22 mmol, yield: 77.8%, purity: 96.9%) was obtained as a yellow solid.

### 1-1-C. Preparation of Compound 4

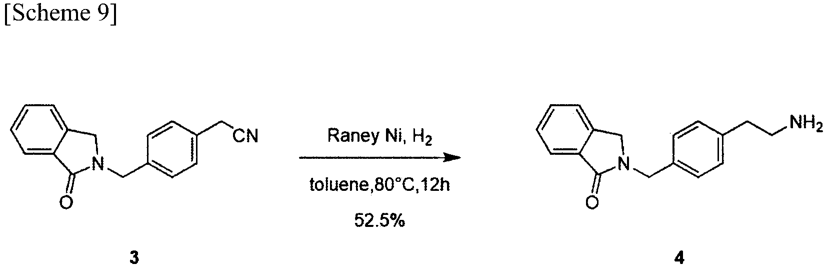

Compound 3 (600 mg, 2.29 mmol, 1.00 eq) and Raney-Ni (19.6 mg, 228 µmol, 0.100 eq) were dissolved in toluene (6.00 mL), degassed, and purged with N₂, and the suspension was then degassed under vacuum, and purged with hydrogen. The resulting mixture was stirred several times in a H₂ (40 psi) environment at 80°C for 12 hours. LC-MS analysis results showed that Compound 3 was completely consumed, and 54.8% of the desired product was detected. The mixture was filtered and concentrated. The crude product was purified by reverse-phase HPLC (column: Phenomenex luna C18 150*40 mm*15 um; mobile phase: [water (FA)-ACN]; gradient: 2% to 32% B over 15 minutes). Compound 4 ([[4-(2-aminoethyl) phenyl] methyl] isoindoli-1-one, 320 mg, 1.20 mmol, yield: 52.5%, purity: 100%) was obtained as a white solid.

### 1-1-D. Preparation of Compound 5

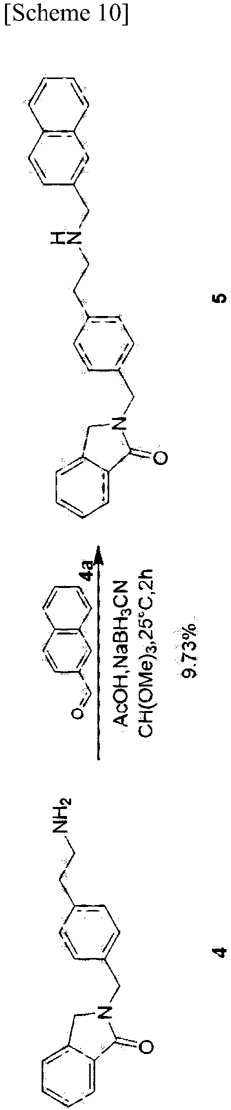

A mixture obtained by dissolving Compound 4 (200 mg, 750 µmol, 1.00 eq) and Compound 4a (117 mg, 750 µmol, 1.00 eq) in CH(OMe)₃ (2.00 mL) was degassed, purged three times with N₂, and stirred at 25°C in N₂ gas for an hour. AcOH (45.0 mg, 750 µmol, 42.9 µL, 1.00 eq) and NaBH₃CN (14.1 mg, 225 µmol, 0.300 eq) were added to the resulting mixture. Thereafter, the mixture was stirred at 25°C in N₂ gas for an hour. TLC (dichloromethane:methanol = 10:1; R_{f} = 0.7) showed that Compound 4 was completely consumed and two new spots were formed. The reaction mixture was concentrated under reduced pressure. The residue was diluted with 5.00 mL of water and extracted with 4.00 mL of EtOAc (4.00 mL * 3). The combined organic layer was washed with 5.00 mL of brine (5.00 mL * 1), dried over Na₂SO₄, filtered, and concentrated under reduced pressure to obtain a residue. The residue was purified by column chromatography (SiO₂, dichloromethane:methanol = 60:1 to 10:1). Compound 5 (34.0 mg, 73.1 µmol, yield: 9.73%, purity: 87.4%) was obtained as a yellow oil.

### 1-1-E. Preparation of Compound 6

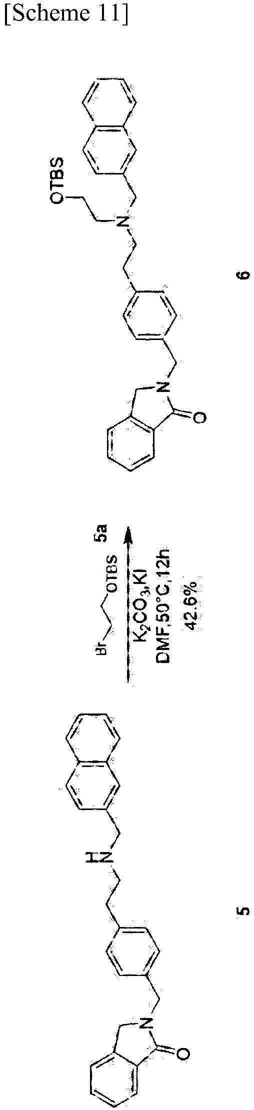

K₂CO₃ (36.7 mg, 265 µmol, 2.00 eq), KI (4.41 mg, 26.5 µmol, 0.200 eq), and Compound 5a (38.1 mg, 159 µmol, 1.20 eq) were added to a mixture obtained by dissolving Compound 5 (54.0 mg, 132 µmol, 1.00 eq) in dimethylformamide (DMF, 0.600 mL). The mixture was stirred at 50°C for 12 hours. TLC (dichloromethane:methanol = 10:1, R_{f} = 0.70) results showed that Compound 5 remained, and one major new spot was detected. The residue was diluted with 2.00 mL of water, and then extracted with 2.00 mL of EtOAc (2.00 mL * 3). The combined organic layer was washed with 3.00 mL of brine (3.00 mL * 1), dried over Na₂SO₄, filtered, and then concentrated under reduced pressure to obtain a residue. The residue was purified by prep-TLC (SiO₂, dichloromethane:methanol = 10:1). Compound 6 (32.0 mg, 56.6 µmol, yield: 42.6%, purity: 100%) was obtained as a yellow oil.

### 1-1-F. Preparation of AON-MG23-05

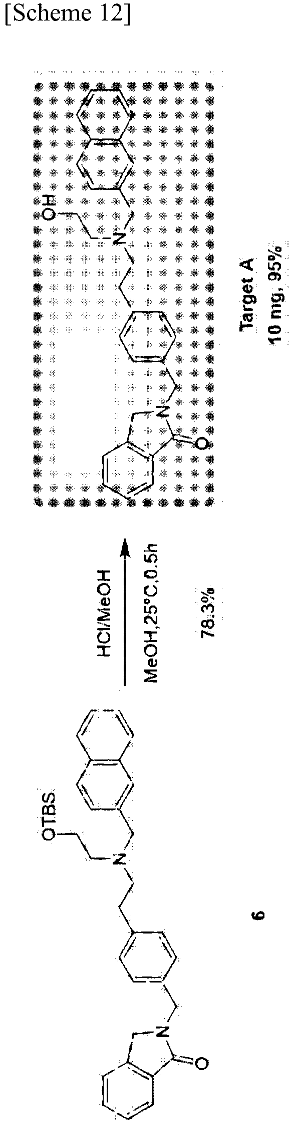

HCl/MeOH (2.00 M, 1.54 mL, 60.0 eq) was added to a mixture obtained by dissolving Compound 6 (29.0 mg, 51.3 µmol, 1.00 eq) in MeOH (0.500 mL). The mixture was stirred at 25°C for 0.5 hours. LC-MS results showed that Compound 6 was completely consumed and 98.7% of the desired mass was detected. The reaction mixture was concentrated under reduced pressure. NaHCO₃ was added to the mixture (pH > 7). The residue was extracted with 3.00 mL of DCM (3.00 mL * 3). The combined organic layer was washed with 2.00 mL of brine (2.00 mL * 1), dried over Na₂SO₄, filtered, and then concentrated under reduced pressure to obtain a residue. The residue was purified by prep-TLC (SiO₂, ethyl acetate:methanol = 10:1). Target A (AON-MG23-05) (18.2 mg, 40.2 µmol, yield: 78.3%, purity: 99.6%) was obtained as an off-white solid.

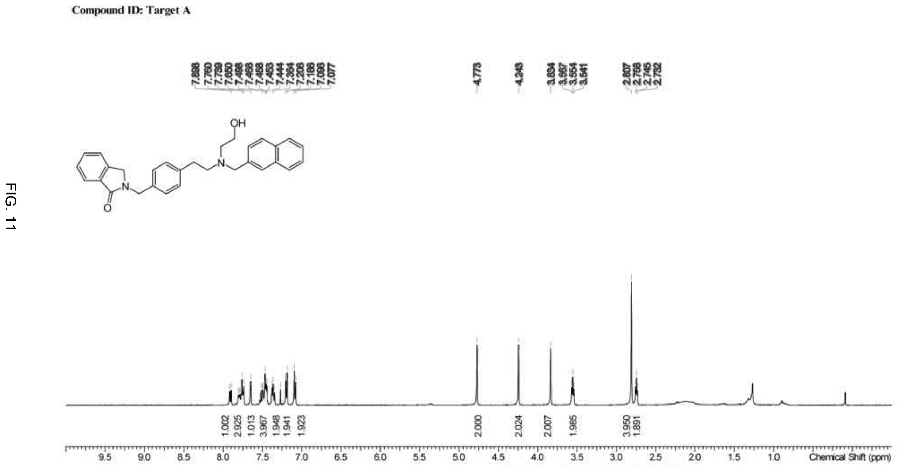

The H NMR data of AON-MG23-05 are shown in FIG. 11.

### 1-2. Preparation of AON-MG23-06

### 1-2-A. Preparation of Compound 8

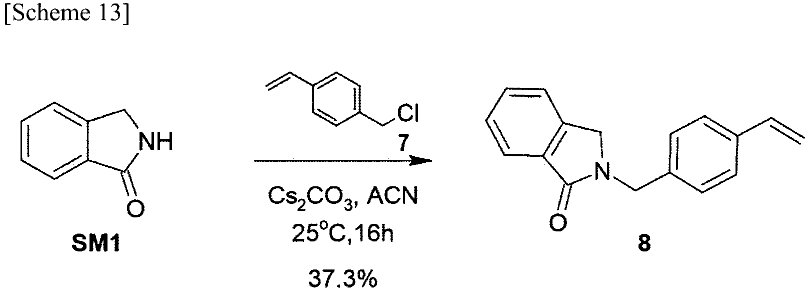

The compound SM1 (3.00 g, 22.5 mmol, 1.00 eq) was mixed with Compound 7 (4.13 g, 27.0 mmol, 3.81 mL, 1.20 eq) and Cs₂CO₃ (11.0 g, 33.8 mmol, 1.50 eq) in ACN (30 mL), and the resulting mixture was degassed, and stirred at 25°C for 16 hours. LC-MS results showed that 20% of the compound SM1 remained. Several new peaks appeared in LC-MS, and 60% of the desired compound was detected. The reaction mixture was quenched by adding 30 mL of H₂O, and extracted with 30 mL of ethyl acetate (EA) (30 mL * 2). The combined organic layer was washed with 30 mL of brine (30 mL * 2), dried over Na₂SO₄, filtered, and then concentrated under reduced pressure to obtain a residue. The residue was purified by column chromatography (SiO₂, petroleum ether:ethyl acetate = 20:1 to 15:1) and TLC (petroleum ether:ethyl acetate = 3:1, R_{f} = 0.43). Compound 8 (2.10 g, 8.42 mmol, yield: 37.3%) was obtained as a white solid, which was confirmed by LC-MS and HNMR.

### 1-2-B. Preparation of Compound 9

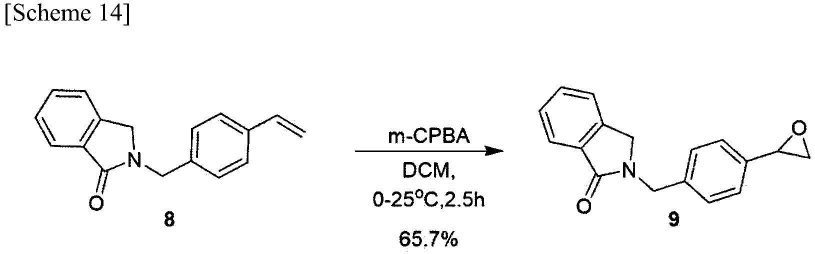

To a mixture obtained by dissolving Compound 8 (200 mg, 802 µmol, 1.00 eq) in dichloromethane (DCM, 0.200 mL), meta-chloroperoxybenzoic acid (m-CPBA, 651 mg, 3.21 mmol, purity: 85.0%, 4.00 eq) was added dropwise at 0°C over 0.5 hours. After this addition, the mixture was stirred at 25°C for 2 hours. TLC (petroleum ether:ethyl acetate = 2:1, R_{f} = 0.33) results showed that Compound 8 was completely consumed and one new spot was formed. The reaction mixture was quenched by adding 20 mL of Na₂S₂O₃ at 25°C, and extracted with 30 mL of DCM (30 mL *2). The combined organic layer was washed with 30 mL of brine (30 mL *2), dried over Na₂SO₄, filtered, and then concentrated under reduced pressure to obtain a residue. The residue was purified by prep-TLC (SiO₂, petroleum ether:ethyl acetate = 2:1). Compound 9 (580 mg, 1.65 mmol, yield: 62.4%) was obtained as a brown solid, which was confirmed by HPLC and HNMR. Also, Compound 9 (140 mg, 527 µmol, yield: 65.7%) was obtained as a yellow oil, which was confirmed by LC-MS and HNMR.

### 1-2-C. Preparation of Compound 9A

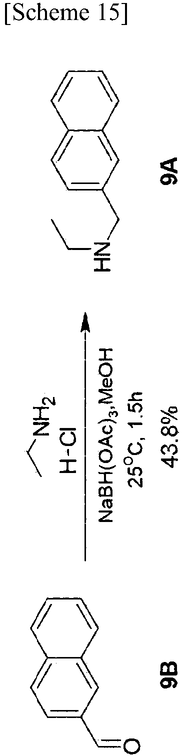

A solution obtained by dissolving ethanamine (626 mg, 7.68 mmol, 909 µL, 1.20 eq, HCl) and triethylamine (TEA, 777 mg, 7.68 mmol, 1.07 mL, 1.2 eq) in MeOH (10 mL) was stirred at 25°C for 0.5 hours, and Compound 9B (1.00 g, 6.40 mmol, 1.00 eq), sodium triacetoxyborohydride (NaBH(OAc)₃, 2.04 g, 9.60 mmol, 1.50 eq), and AcOH (3.85 mg, 64.0 µmol, 3.67 µL, 0.01 eq) were added at 25°C. The resulting mixture was stirred at 25°C for an hour. LC-MS results showed that Compound 9B was completely consumed, and one major peak having a desired m/z value was detected. The reaction mixture was quenched by adding 10 mL of Na₂CO₃, diluted with 20 mL of H₂O, and then extracted with 30 mL of EA (30 mL * 2). The combined organic layer was washed with 30 mL of brine (30 mL * 1), dried over Na₂SO₄, filtered, and then concentrated under reduced pressure to obtain a residue. The residue was purified by column chromatography (SiO₂, petroleum ether:ethyl acetate = 0:1 to dichloromethane:methanol = 20:1). Compound 9A (520 mg, 2.81 mmol, yield 43.8%) was obtained as a white solid, which was confirmed by HNMR and LC-MS.

### 1-2-D. Preparation of AON-MG23-06

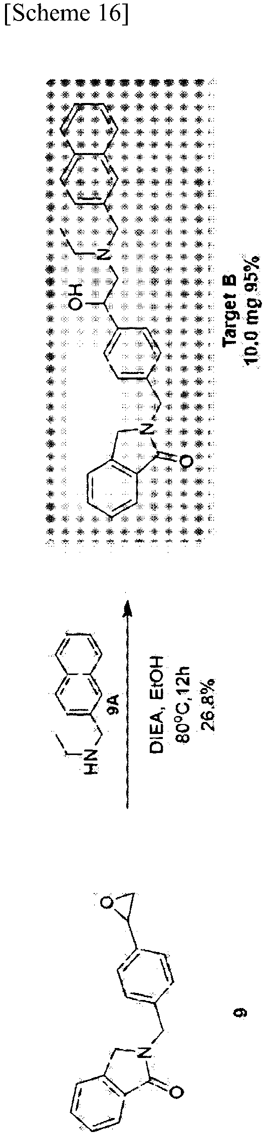

A mixture obtained by dissolving Compound 9 (65.0 mg, 245 µmol, 1.00 eq), Compound 9A (54.4 mg, 294 µmol, 1.20 eq), and N,N-diisopropylethylamine (DIPEA, 94.9 mg, 735 µmol, 128 µL, 3.00 eq) in ethanol (0.1 mL) was degassed, purged three times with N₂, and then stirred at 80°C in N₂ gas for 12 hours. LC-MS results showed that Compound 9 was completely consumed, and one major peak having a desired m/z value was detected. The reaction mixture was quenched by adding 10 mL of H₂O, extracted with 5 mL of EA (5 mL * 2), and then concentrated under reduced pressure to obtain a residue. The residue was purified by prep-HPLC (FA conditions: Phenomenex luna C18 150 * 25 mm * 10 µm; mobile phase: [water (FA)-ACN]; gradient: 15% to 45% B, ≥1 minute). Subsequently, it was purified by prep-HPLC (neutral conditions: Waters Xbridge 150*25 mm*5 µm; mobile phase: [water (NH₄HCO₃)-ACN]; gradient: 45% to 75% B, ≥15 minutes). AON-MG23-06 (Target B) (29.7 mg, 65.7 µmol, yield: 26.8%, purity: 99.8%) was obtained as a white solid, which was confirmed by LC-MS, HPLC, H NMR, and special NMR.

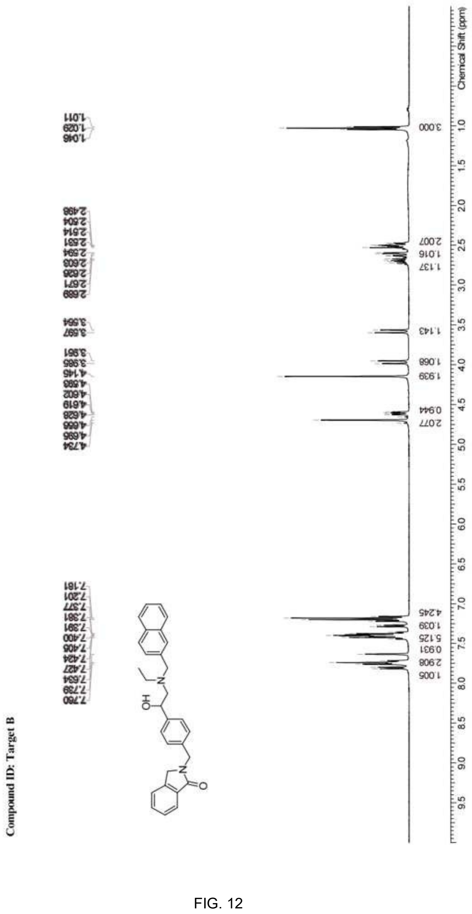

The H NMR data of AON-MG23-06 are shown in FIG. 12.

### 1-3. Preparation of AON-MG23-07

### 1-3-A. Preparation of Compound 10

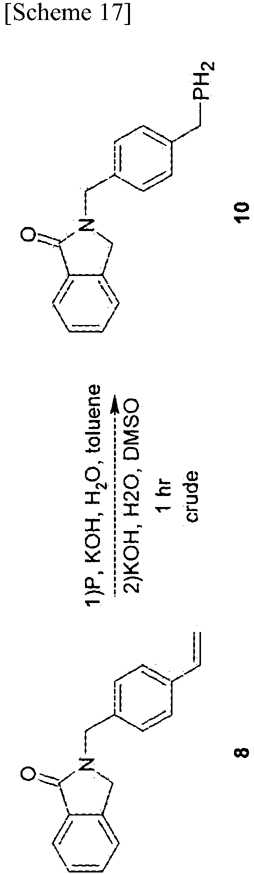

A suspension of KOH (675.1 mg, 12.0 mmol, 3.00 eq), dimethyl sulfoxide (DMSO, 10.0 mL), toluene (7.50 mL), and H₂O (2.00 mL) was purged with argon, and saturated with P (5.00 g, 147.0 mmol, 36.6 eq). The DMSO solution containing Compound 8 (1.00 g, 4.01 mmol, 1.00 eq) was stirred at 70°C for 30 minutes, and continuously added dropwise to allow an ignition gas to pass therethrough. The reaction mixture was further heated (70°C) for 30 minutes while maintaining a flow of phosphine. TLC (PE:EA = 3:1, R_{f} = 0.38) results showed that Compound 8 was completely consumed and a new spot was formed. The mixture was passed through argon, cooled, diluted with 20 mL of water, and then extracted with toluene (10 mL*2). The toluene extract was washed with brine (10 mL*1), dried over Na₂SO₄, filtered, and then concentrated under reduced pressure to obtain a residue. Compound 10 (1.10 g, crude) was obtained as a white solid.

### 1-3-B. Preparation of AON-MG23-07

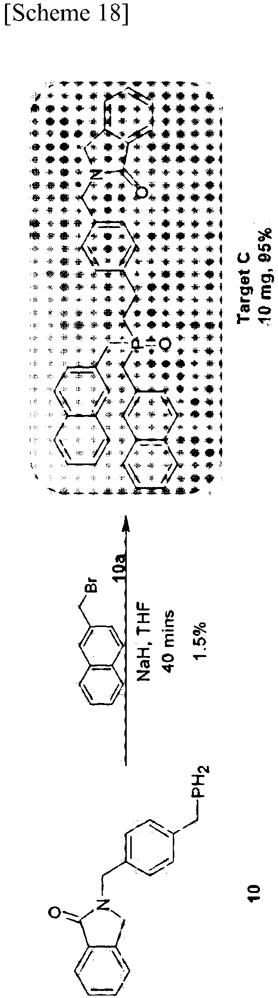

To a solution obtained by dissolving Compound 10 (1.10 g, 3.88 mmol, 1.00 eq) in tetrahydrofuran (THF, 10.0 mL), NaH (465.9 mg, 11.6 mmol, purity: 60.0%, 3.00 eq) was added at 0°C under a N₂ gas atmosphere. The mixture was stirred at 0°C for 30 minutes. Thereafter, a THF solution containing Compound 10a (858.4 mg, 3.88 mmol, 1.00 eq) was added dropwise at 0°C. The mixture was stirred at 0°C for 10 minutes. LC-MS results showed that Compound 10 was completely consumed and the desired mass was detected. The reaction mixture was quenched by adding 20 mL of NH₄Cl in N₂ gas at 0°C, diluted with 20.0 mL of H₂O, and extracted with 40.0 mL of EA (20.0 mL * 2). The combined organic layer was washed with 30.0 mL of brine (30.0 mL * 1), dried over Na₂SO₄, filtered, and then concentrated under reduced pressure to obtain a residue. The residue was purified by prep-HPLC (FA conditions; column: Phenomenex luna C18 150 * 40 mm * 15 um; mobile phase: [water (FA)-ACN]; gradient: 52% to 82% B over 15 min). The residue was further purified by prep-HPLC (FA conditions; column: Welch Xtimate C18 150 * 25 mm * 5 µm; mobile phase: [water (FA)-ACN]; gradient: 58% to 78% B for 15 min). Off-white AON-MG23-07 (Target C) (35.0 mg, 58.5 µmol, yield: 1.51%, purity: 97.0%) was obtained.

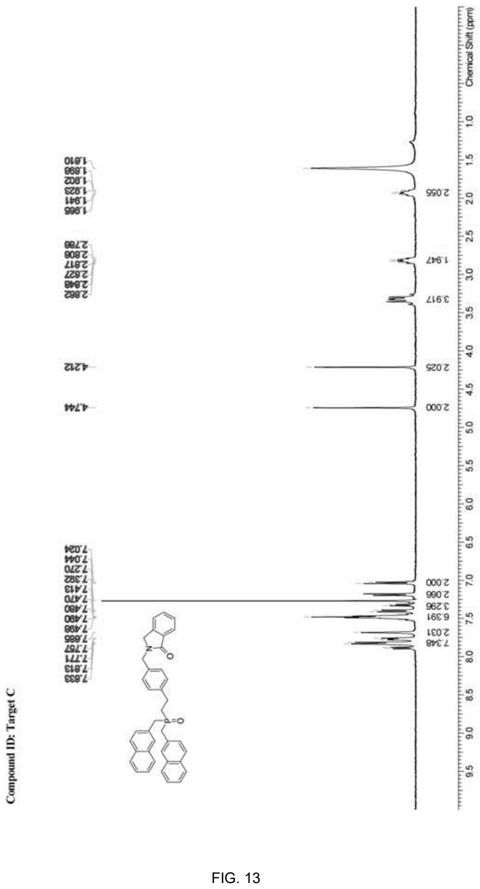

The H NMR data of AON-MG23-07 are shown in FIG. 13.

### 1-4. Preparation of AON-MG23-08

### 1-4-A. Preparation of Compound 2

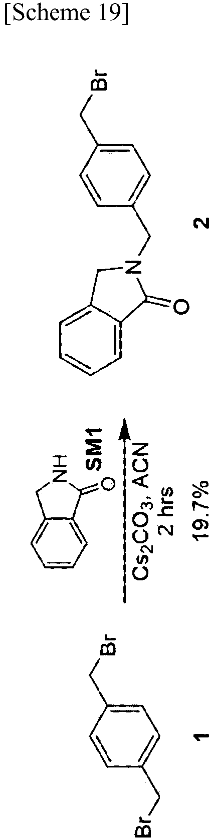

A mixture obtained by dissolving Compound 1 (5.00 g, 18.9 mmol, 1.00 eq), the compound SM1 (1.26 g, 9.47 mmol, 0.50 eq), and Cs₂CO₃ (4.94 g, 15.1 mmol, 0.80 eq) in ACN (15.0 mL) was degassed, treated three times with N₂ gas, and stirred at 80°C in N₂ gas for 2 hours. LC-MS (EW47929-30-P1A1) results showed that the compound SM1 was completely consumed and the desired mass was detected. The reaction mixture was diluted with 30.0 mL of H₂O, and extracted with 40.0 mL of EA (20.0 mL * 2). The combined organic layer was washed with 30.0 mL of brine (30.0 mL * 1), dried over Na₂SO₄, filtered, and then concentrated under reduced pressure to obtain a residue. The residue was purified by column chromatography (SiO₂, petroleum ether/ethyl acetate = 20/1 to 5/1, TLC, PE:EA = 3:1, R_{f} = 0.22). Compound 2 (1.18 g, 3.73 mmol, yield: 19.7%) was obtained as a yellow solid.

### 1-4-B. Preparation of Compound 3

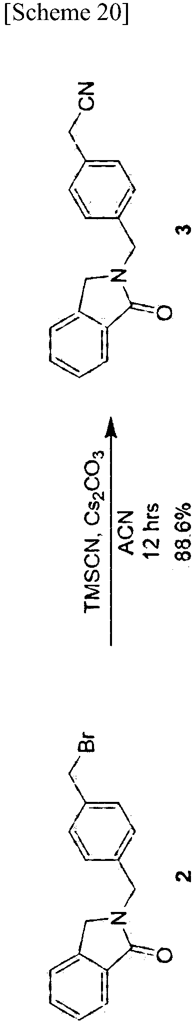

Cs₂CO₃ (1.36 g, 4.17 mmol, 1.20 eq) and TMSCN (414.1 mg, 4.17 mmol, 522 µL, 1.20 eq) were added to a solution obtained by dissolving Compound 2 (1.1 g, 3.48 mmol, 1.00 eq) in ACN (16.0 mL). The mixture was stirred at 25°C for 12 hours. LC-MS analysis results showed that Compound 2 was completely consumed and the desired mass was detected. The reaction mixture was concentrated under reduced pressure to remove the solvent. The residue was diluted with 20.0 mL of H₂O, and extracted with 40.0 mL of EA (20.0 mL * 2). The combined organic layer was washed with 20.0 mL of brine, dried over Na₂SO₄, filtered, and then concentrated under reduced pressure to obtain a residue. The residue was purified by column chromatography (SiO₂, petroleum ether/ethyl acetate = 5/1 to 3/1, PE: EA = 5:1, R_{f} = 5:1, R_{f} = 0.47). Compound 3 (825 mg, 3.08 mmol, yield: 88.6%, purity: 98.0%) was obtained as a yellow solid.

### 1-4-C. Preparation of Compound 4

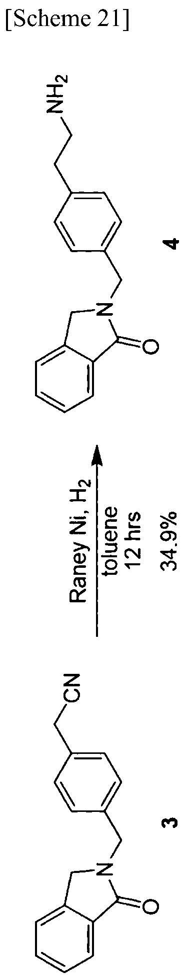

Compound 3 (825 mg, 3.15 mmol, 1.00 eq) and Raney-Ni (26.9 mg, 314 µmol, 0.10 eq) were mixed in toluene (8.00 mL), and the resulting mixture was treated under vacuum and purged three times with N₂, and then purged several times with H₂ under vacuum. The mixture was stirred at 80°C under a H₂ (40 psi) atmosphere for 12 hours. LC-MS results showed that Compound 3 was completely consumed and the desired mass was detected. The reaction mixture was filtered and concentrated under reduced pressure to obtain a residue. Thereafter, the residue was purified by prep-HPLC (FA conditions; column: Phenomenex luna C18 150 * 40 mm *15 µm; mobile phase: [water (FA)-ACN]; gradient: 8% to 38% B, for 15 min). Compound 4 (300 mg, 1.10 mmol, yield: 34.9%, purity: 97.5%) was obtained as a white solid, which was confirmed by HNMR and LC-MS.

### 1-4-D. Preparation of Compound 5

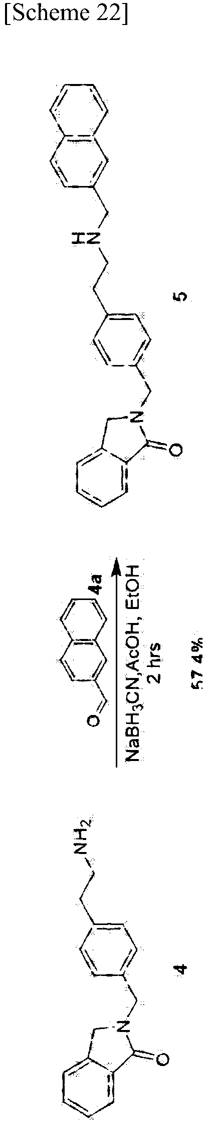

Compound 4a (175 mg, 1.13 mmol, 1.00 eq) was added to a solution obtained by dissolving Compound 4 (300 mg, 1.13 mmol, 1.00 eq) in 5.00 mL of ethanol (EtOH). The mixture was stirred at 25°C for an hour under a N₂ atmosphere. Thereafter, NaBH₃CN (42.4 mg, 675 µmol, 0.60 eq) and AcOH (67.6 mg, 1.13 mmol, 64.4 µL, 1.00 eq) were added at 25°C, and stirred for an hour under a N₂ atmosphere. LC-MS results showed that 2% of Compound 4 was completely consumed and the desired mass was detected. The residue was diluted with 10 mL of H₂O and extracted with 20 mL of EA (10 mL * 2). The organic phases were combined, washed with 10 mL of brine (10 mL * 1), dried over Na₂SO₄, filtered, and then concentrated under reduced pressure to obtain a residue. Then, the residue was purified by column chromatography (SiO₂, DCM/MeOH = 20/1 to 15/1), TLC, DCM:MeOH = 10:1, R_{f} = 0.46). Compound 5 (293 mg, 647 µmol, yield: 57.4%, purity: 100%, FA) was obtained as a white solid, which was confirmed by LC-MS and H NMR.

### 1-4-E. Preparation of AON-MG23-08

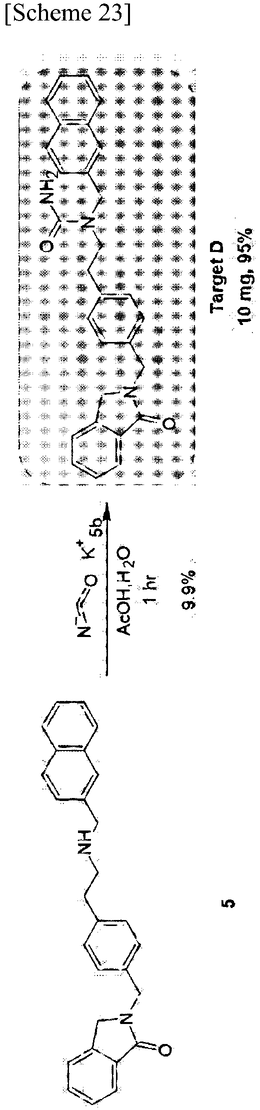

Compound 5b (99.7 mg, 1.23 mmol, 48.5 µL, 5.00 eq) and H₂O (1.50 mL) were added to a solution obtained by dissolving Compound 5 (100 mg, 245 µmol, 1.00 eq) in AcOH (0.30 mL). The mixture was stirred at 25°C for an hour. LC-MS analysis results showed that approximately 10% of Compound 5 remained and the desired mass was detected. The reaction was stopped by slowly adding a saturated sodium hydroxide solution until the pH reached 8, and the mixture was then extracted with 40 mL of DCM (20 mL * 2). The combined organic layer was washed with 40.0 mL of brine (20.0 mL * 2), dried over Na₂SO₄, filtered, and then concentrated under reduced pressure to obtain a residue. The residue was purified by prep-TLC (SiO₂, DCM:MeOH = 15:1). The residue was purified by prep-HPLC (basic conditions; column: Waters Xbridge 150 * 25 mm * 5 um; mobile phase: [water (ammonia hydroxide v/v) - ACN]; gradient: 25% to 55% B, over 10 min). AON-MG23-08 (Target D) (11.0 mg, 24.4 µmol, yield: 95%, purity: 100%) was obtained as a white solid.

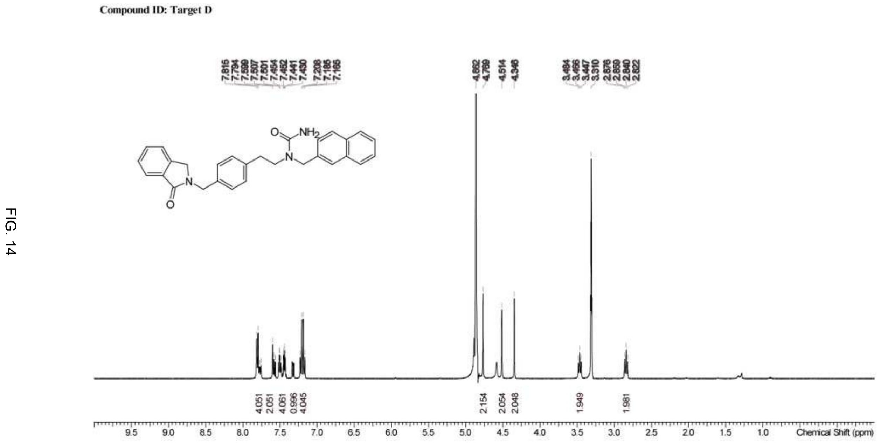

The H NMR data of AON-MG23-08 are shown in FIG. 14.

Derivatives of AON-MG23-02 were prepared in the same manner as described above, and their structural formulas are shown in Table 2 below.

**[Table 2]**

| Classification | Name | Structure |
|---|---|---|
| AON-MG23-05 | 2-(4-(2-((2-hydroxyethyl)(naphthalen-2-yl methyl)amino)ethyl)benzyl)isoindolin-1-one | 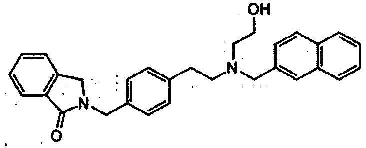 |
| AON-MG23-06 | 2-(4-(2-(ethyl(naphthalen-2-ylmethyl)ami no)-1-hydroxyethyl)benzyl)isoindolin-1-one | 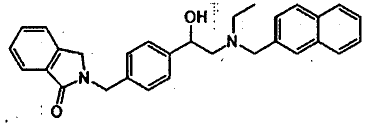 |
| AON-MG23-07 | 2-(4-(2-(bis(naphthalen-2-ylmethyl)phos phoryl)ethyl)benzyl)isoindolin-1-one | 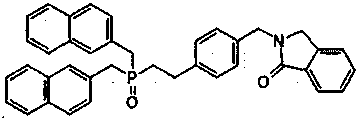 |
| AON-MG23-08 | 1-(naphthalen-2-ylmethyl)-1-(4-((1-oxois oindolin-2-yl)methyl)phenethyl)urea | 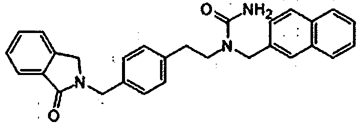 |

### 2. Comparison of inhibitory effects of four derivatives of AON-MG23-02 on ANO1 and EGFR protein expression in glioblastoma cell line

The inhibitory effects of AON-MG23-02 derivatives AON-MG23-05, AON-MG23-06, AON-MG23-07, and AON-MG23-08 on ANO1 and EGFR protein expression in a glioblastoma cell line were compared. A human glioblastoma cell line U87-MG was seeded at a density of 100 X 10⁴ cells/dish in a 60 mm dish, and cultured for 24 hours. Thereafter, the cells were treated with 0, 8, 16, and 32 µM of the AON-MG23-05, AON-MG23-06, AON-MG23-07, and AON-MG23-08 derivatives, respectively, and cultured for 48 hours. The cultured cells were harvested, and the harvested cells were lysed for Western blotting. An anti-ANO1 antibody (Abcam, ab53212), an anti-pEGFR antibody (Cell Signaling, #4407S), an anti-EGFR antibody (Cell Signaling, #4267S), and an anti-ACTIN antibody (sigma, #A2066) were used as antibodies for Western blotting. Protein concentration was quantified using the BSA method (a protein quantitation assay, a bovine serum albumin assay) (Pierce, Cat. 23225). After the cells were lysed, an equal amount (10 µg) of the proteins obtained from the lysed cells was subjected to 10% SDS-PAGE to separate the proteins depending on their molecular weights, transferred to a PVDF membrane (Bio-rad), and treated with a blocking buffer (5% skim milk in a Tris-buffered saline (TBS) buffer containing 0.1% Tween 20, TBS-T) at room temperature for an hour. Then, the PVDF membrane was treated with the primary antibody and reacted at 4°C for 16 hours. The reacted PVDF membrane was washed three times with TBS-T for 10 minutes. The PVDF membrane was then reacted with the horseradish peroxidase-labeled secondary antibody at room temperature for an hour, and then washed three times with TBS-T for 10 minutes. The washed PVDF membrane was treated with the ECL kit (Thermo, West Pico Plus), reacted, and visualized using the da Vinci-Q (Youngin Lab Plus) device. The amount of each protein was quantified and analyzed using Image J software.

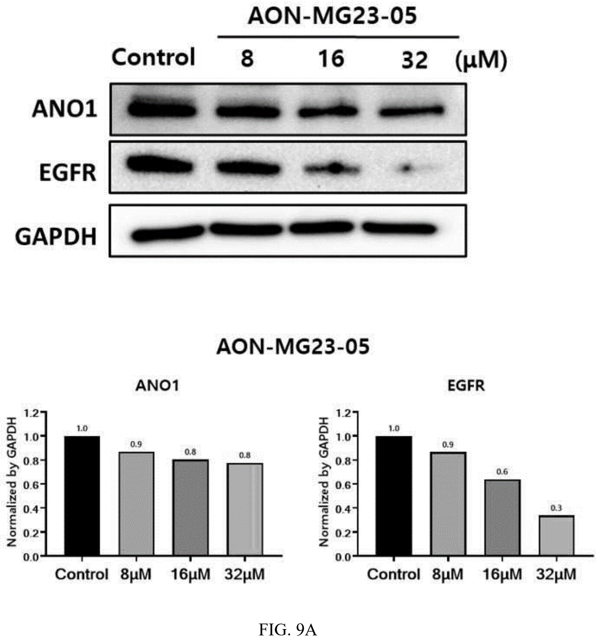

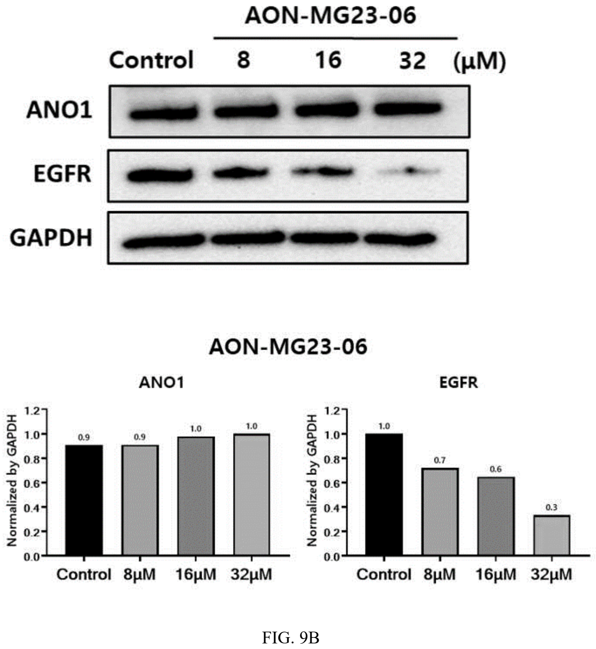

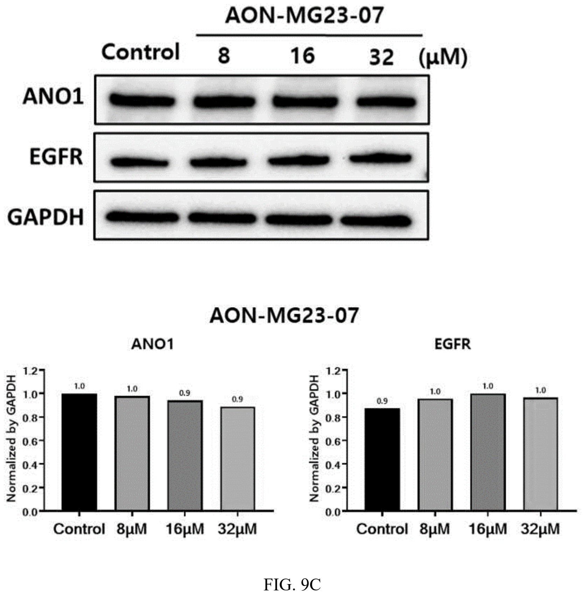

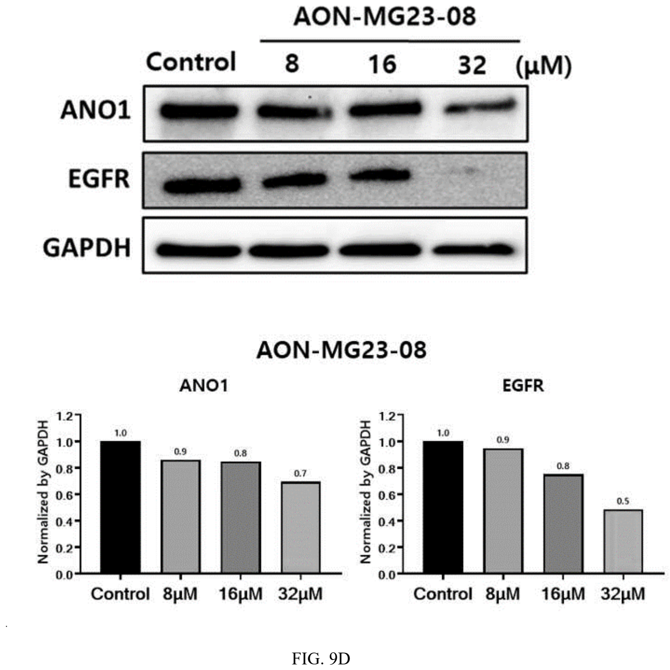

As a result, as shown in FIGS. 9A to 9D, it was confirmed that the derivatives AON-MG23-05 and AON-MG23-08 had reduced expression of ANO1 and EGFR proteins compared to the untreated control.

### 3. Comparison of metastasis inhibitory effects of four derivatives of AON-MG23-02 in human glioblastoma

A human glioblastoma cell line U87-MG was seeded at a density of 8 X 10⁴ cells/well on the top of a Matrigel-coated filter (Transwell invasion chambers, Corning), and then treated with 0, 8, 16, and 32 µM of each of the compound derivatives AON-MG23-05, AON-MG23-06, AON-MG23-07, and AON-MG23-08 of the present invention, and cultured at 37°C in a CO₂ incubator for 24 hours. After the culturing was completed, the cells were fixed and stained using a Diff-Quick stain kit (Sysmex, Kobe, Japan). Images of the stained cells were taken under a microscope, the cells were counted using Image J software, and statistical analysis was then performed.

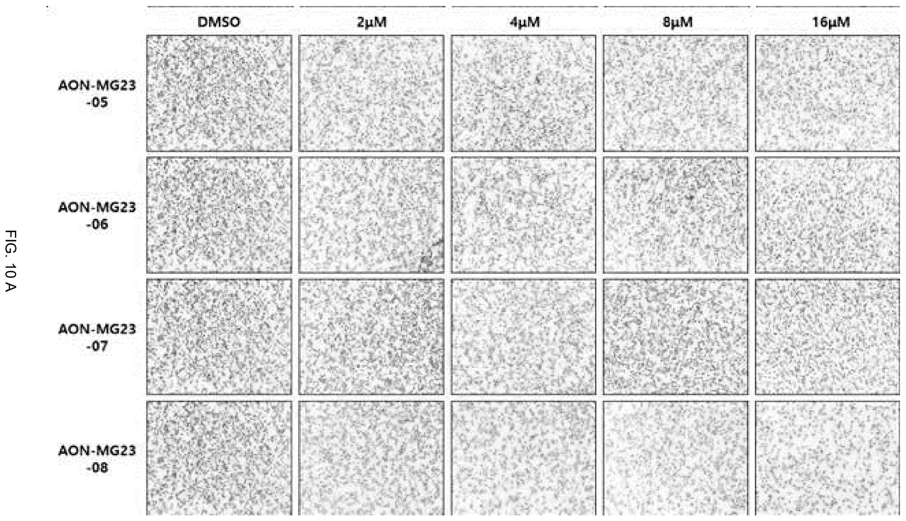

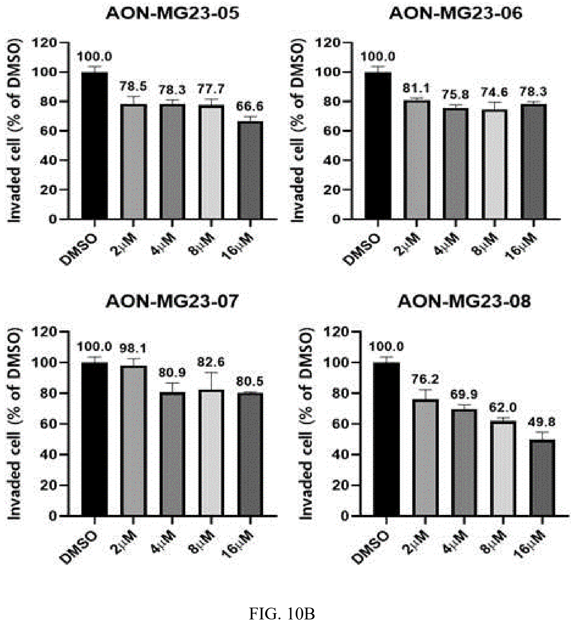

As a result, as shown in FIGS. 10A AND 10B, it can be seen that the groups treated with the derivatives AON-MG23-05, AON-MG23-06, AON-MG23-07, and AON-MG23-08 showed a reduced degree of penetration into Matrigel compared to the untreated control, indicating that the derivatives inhibit metastasis in human glioblastoma.

The foregoing description of the present invention is provided for illustrative purposes only, and it will be understood by those of ordinary skill in the art to which the invention pertains that various modifications can be made in other specific forms without departing from the spirit or essential characteristics of the invention. Therefore, the embodiments described above are to be considered in all respects as illustrative and not restrictive.

### [Industrial Applicability]

The present invention relates to the use of novel Anoctamin 1 (ANO1) inhibitory compounds for the prevention and treatment of brain tumors. The compounds of the present invention are capable of simultaneously inhibiting ANO1 and EGFR in brain tumor cells, and may not only exhibit a more potent anticancer effect through the dual inhibition of these two proteins, but also enhance the efficacy of anticancer agents, such as EGFR-targeted anticancer drugs, and suppress drug resistance when used in combination therewith. Therefore, the compounds may be used as dual-target anticancer agents against ANO1 and EGFR by themselves, and may also be utilized as combination agents for EGFR-targeted therapy, and thus are expected to be useful in various applications in the prevention and treatment of brain tumors and to have industrial applicability.

## Claims

1. A pharmaceutical composition for preventing or treating brain tumors, comprising, as an active ingredient, at least one compound selected from the group consisting of compounds represented by Chemical Formulae 1 to 6, or pharmaceutically acceptable salts thereof:

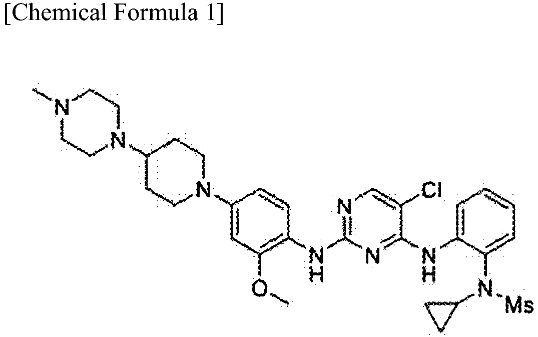

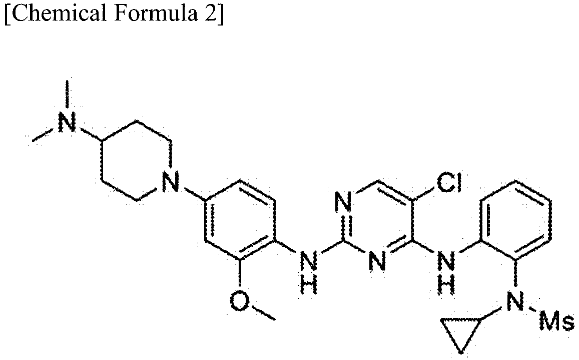

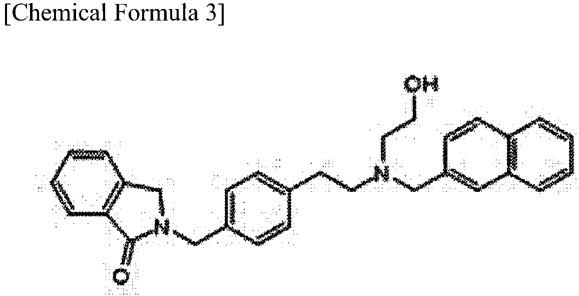

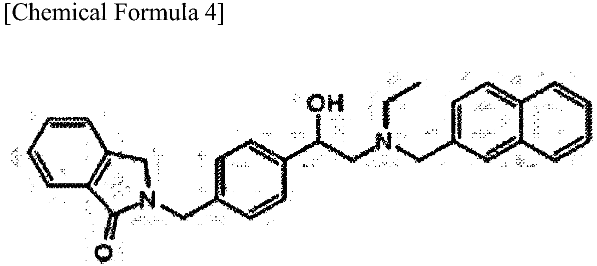

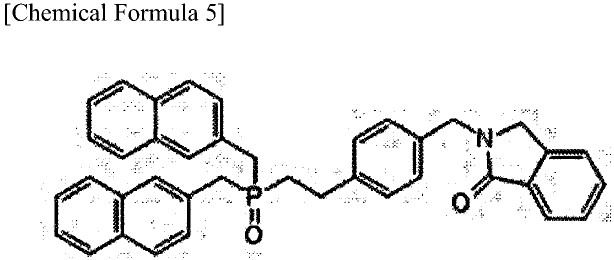

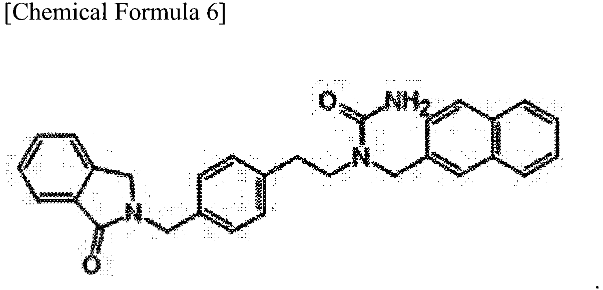

2. The pharmaceutical composition of claim 1, wherein the brain tumors are associated with at least one mutation selected from the group consisting of EGFR (epidermal growth factor receptor) and ANO1 (Anoctamin 1).

3. The pharmaceutical composition of claim 1, wherein the compound or pharmaceutically acceptable salts thereof simultaneously inhibits EGFR and ANO1.

4. The pharmaceutical composition of claim 1, wherein the compound or pharmaceutically acceptable salts thereof satisfies at least one property selected from the group consisting of:
(a) inhibiting tumor metastasis; and
(b) suppressing resistance of cancer to an anticancer agent.

5. The pharmaceutical composition of claim 4, wherein the anticancer agent is a targeted anticancer agent against a tyrosine kinase.

6. The pharmaceutical composition of claim 5, wherein the tyrosine kinase is one or more selected from the group consisting of EGFR (epidermal growth factor receptor), ALK (anaplastic lymphoma kinase), ROS1 (ROS Proto-Oncogene 1), BRAF (B-Raf Proto-Oncogene), HER2 (human epidermal growth factor receptor 2), RET (Ret Proto-Oncogene), NTRK1 (Neurotrophic Receptor Tyrosine Kinase 1), MET (Mesenchymal-Epithelial Transition factor), and NRG1 (Neuregulin 1).

7. The pharmaceutical composition of claim 1, wherein the pharmaceutical composition is administered in combination with a targeted anticancer agent against a tyrosine kinase.

8. The pharmaceutical composition of claim 7, wherein the pharmaceutical composition is administered simultaneously, separately, or sequentially with the targeted anticancer agent.

9. A pharmaceutical composition for the prevention or treatment of brain tumors, comprising as active ingredients:
(i) at least one compound selected from the group consisting of compounds represented by Chemical Formulae 1 to 6, or pharmaceutically acceptable salts thereof, and
(ii) a targeted anticancer agent against EGFR.

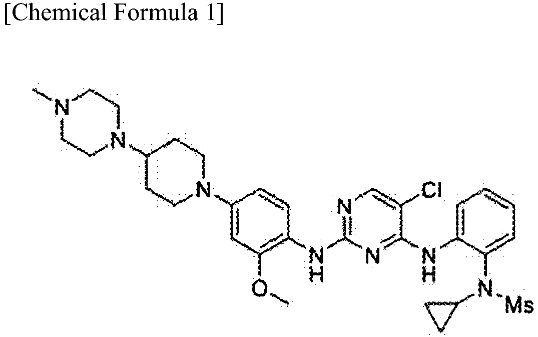

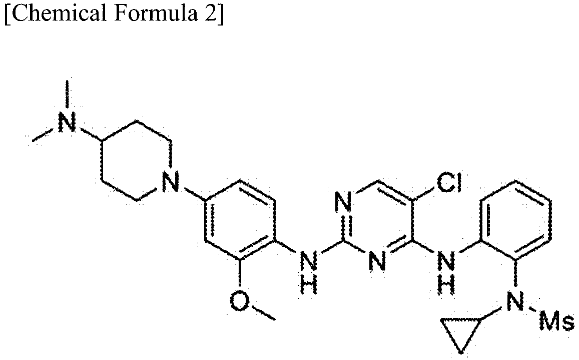

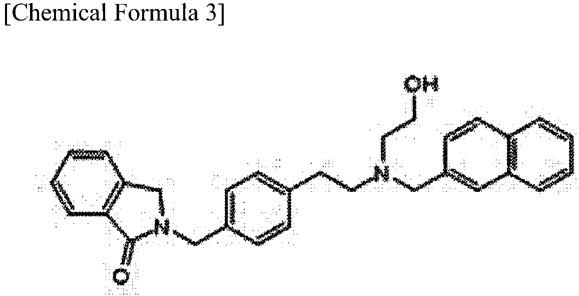

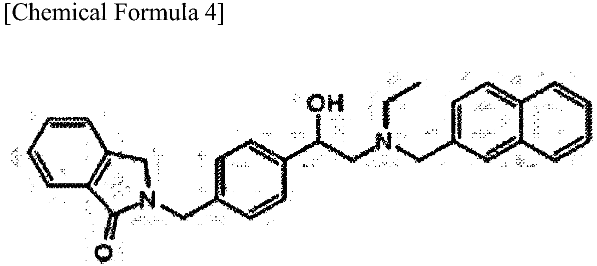

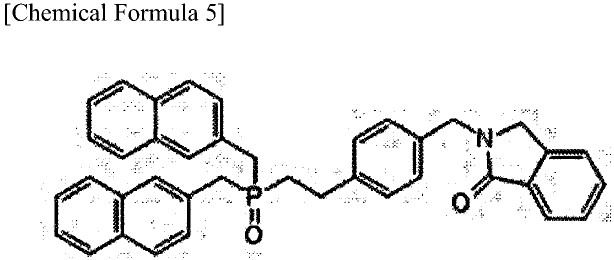

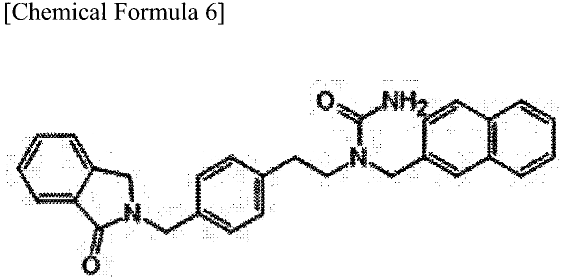

10. The pharmaceutical composition of claim 9, wherein the compound or pharmaceutically acceptable salts thereof inhibits resistance of brain tumor cells to the targeted anticancer agent against EGFR.

11. The pharmaceutical composition of claim 9, wherein the composition is in the form of a mixed formulation in which the compound or pharmaceutically acceptable salts thereof and the targeted anticancer agent against EGFR are mixed together.

12. The pharmaceutical composition of claim 9, wherein the composition is in a form in which the compound or pharmaceutically acceptable salts thereof and the targeted anticancer agent against EGFR are separately formulated and administered simultaneously, separately, or sequentially.

13. A pharmaceutical composition for enhancing the anticancer effect of a brain tumor anticancer agent, comprising, as an active ingredient, at least one compound selected from the group consisting of compounds represented by Chemical Formulae 1 to 6 or pharmaceutically acceptable salts thereof:

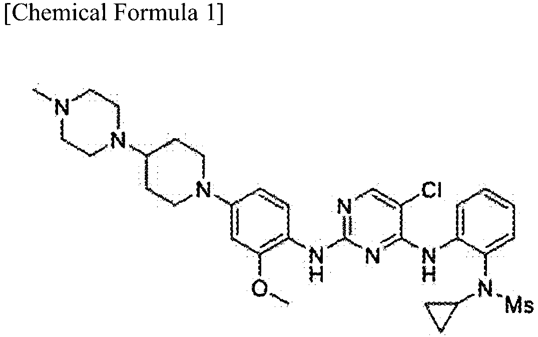

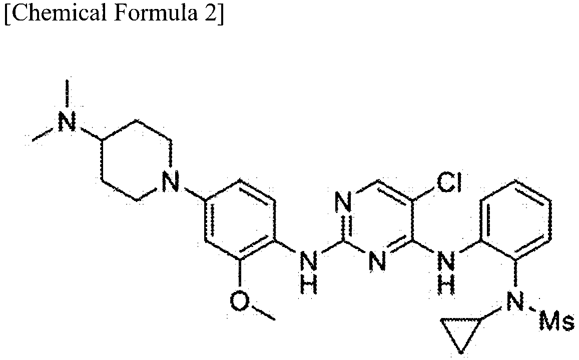

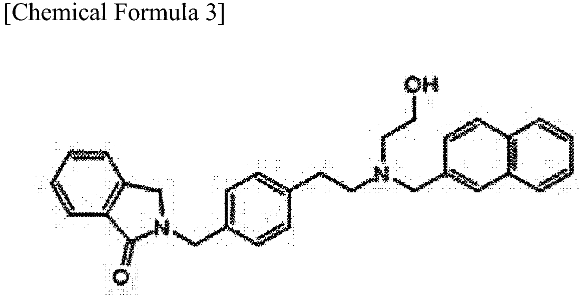

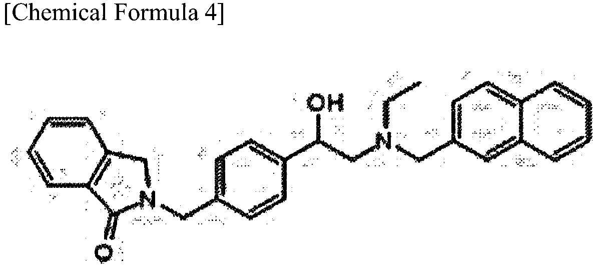

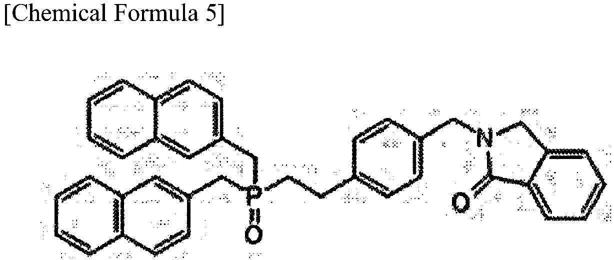

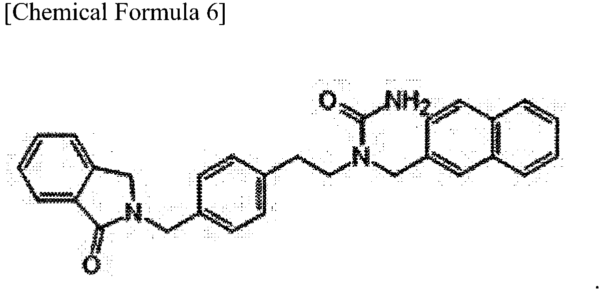

14. The pharmaceutical composition of claim 13, wherein the brain tumor anticancer agent is a targeted anticancer agent against a tyrosine kinase.

15. The pharmaceutical composition of claim 14, wherein the tyrosine kinase is one or more selected from the group consisting of EGFR (epidermal growth factor receptor), ALK (anaplastic lymphoma kinase), ROS1 (ROS Proto-Oncogene 1), BRAF (B-Raf Proto-Oncogene), HER2 (human epidermal growth factor receptor 2), RET (Ret Proto-Oncogene), NTRK1 (Neurotrophic Receptor Tyrosine Kinase 1), MET (Mesenchymal-Epithelial Transition factor), and NRG1 (Neuregulin 1).

16. The pharmaceutical composition of claim 13, wherein the composition is administered simultaneously, separately, or sequentially with the brain tumor anticancer agent.

17. A method for preventing or treating brain tumors, comprising administering, to a subject in need thereof, at least one compound selected from the group consisting of the compounds represented by Chemical Formulae 1 to 6 or pharmaceutically acceptable salts thereof, or a composition comprising the same as an active ingredient.

18. A use of at least one compound selected from the group consisting of the compounds represented by Chemical Formulae 1 to 6 or pharmaceutically acceptable salts thereof, or a composition comprising the same as an active ingredient, for the prevention or treatment of brain tumors.

19. A use of at least one compound selected from the group consisting of the compounds represented by Chemical Formulae 1 to 6 for the manufacture of a medicament for treating brain tumors.

20. A method for preventing or treating brain tumors, comprising administering, to a subject in need thereof, (i) at least one compound selected from the group consisting of the compounds represented by Chemical Formulae 1 to 6 or pharmaceutically acceptable salts thereof, and (ii) a targeted anticancer agent against EGFR.

21. A use of a composition comprising as active ingredients, (i) at least one compound selected from the group consisting of the compounds represented by Chemical Formulae 1 to 6 or pharmaceutically acceptable salts thereof, and (ii) a targeted anticancer agent against EGFR, for preventing or treating brain tumors.

22. A use of a composition comprising as active ingredients, (i) at least one compound selected from the group consisting of the compounds represented by Chemical Formulae 1 to 6 or pharmaceutically acceptable salts thereof, and (ii) a targeted anticancer agent against EGFR, for the manufacture of a medicament for treating brain tumors.

23. A method for enhancing the anticancer effect of an anticancer agent for brain tumors,
comprising administering, to a subject in need thereof, at least one compound selected from the group consisting of the compounds represented by Chemical Formulae 1 to 6 or pharmaceutically acceptable salts thereof, or a composition comprising the same as an active ingredient.

24. A use of at least one compound selected from the group consisting of the compounds represented by Chemical Formulae 1 to 6 or pharmaceutically acceptable salts thereof, or a composition comprising the same as an active ingredient, for enhancing the anticancer effect of an anticancer agent for brain tumors.

25. A use of at least one compound selected from the group consisting of the compounds represented by Chemical Formulae 1 to 6 for the manufacture of a medicament for enhancing the anticancer effect of an anticancer agent for brain tumors.
